# EUROPEAN PATENT APPLICATION

(11) **EP 4 198 512 A1**
(43) Date of publication of application: **21.06.2023**
(21) Application number: 21856250.2
(22) Date of filing: 12.08.2021
(51) Int. Cl.: G01N 33/68, G01N 33/58, C12Q 1/25, A61K 47/64, A61P 35/00

(54) **COLON CANCER DIAGNOSIS OR DETECTION COMPOSITION FOR PREDICTING RESPONSE TO DRUG, AND COMPOSITION FOR PREVENTING OR TREATING COLON CANCER**

(30) Priority: 14.08.2020 KR 20200102142
(71) Applicant: Korea Institute of Science and Technology, Seoul 02792 (KR); Korea University Research and Business Foundation, Seoul 02841 (KR)
(72) Inventor: RYU, Ju Hee, Seoul 02792 (KR); KWON, Ick Chan, Seoul 02792 (KR); KIM, Kwangmeyung, Seoul 02792 (KR); KIM, Han Young, Seoul 02792 (KR); KIM, Eun-sun, Seoul 06289 (KR)
(74) Representative: advotec.
(86) International application number: PCT/KR2021/010704
(87) International publication number: WO 2022/035244

(57) **Abstract**

The present disclosure relates to a cancer cell-specific complex for targeting cancer. The complex for targeting cancer according to the present disclosure includes EGF, but is affected more by lysosomal activity rather than the EGFR signaling pathway, and thus can overcome the shortcomings of the existing EGF therapy-based diagnostic and therapeutic compositions and provide successfully personalized and improved effects of treating, preventing and alleviating cancer.

A diagnostic composition according to the present disclosure enables the prediction of the anticancer performance of a therapeutic composition of the present disclosure in a subject, and thus enables anticancer treatment to be designed stably.

## Description

### [Technical Field]

The present disclosure relates to a composition for preventing or treating colon cancer for predicting drug response, more particularly to a composition that can specifically diagnose or detect epidermal growth factor receptor (EGFR)-positive colon cancer cells by penetrating into the epidermal growth factor receptor (EGFR)-positive colon cancer cells and can effectively prevent or treat epidermal growth factor receptor (EGFR)-positive colon cancer through binding with a drug.

### [Background Art]

According to the statistics of the International Agency for Research on Cancer of the World Health Organization, Korea is the number one in the incidence of colon cancer. With population aging, obesity, change of diet, etc., colon cancer is among top cancers occurring in Koreans. For treatment of colon cancer, various chemotherapeutic agents, immunotherapeutic agents and targeted therapeutic agents have been developed. Among them, it is known that the targeted therapeutic agents exhibit superior effect of specifically targeting colon cancer cells and inhibiting their growth or killing them. However, the targeted therapeutic agents show very different therapeutic responses depending on the patients' genetic characteristics. Actually, although anti-EGFR drugs such as cetuximab (Erbitux^{™}) are known as the effective number one therapy for colon cancer, only some patients show response to the anti-EGFR drugs. Furthermore, although the anti-EGFR therapy is very expensive, the anti-EGFR drugs are clinically administered to many patients because it is difficult to predict drug response and efficacy is achieved in only a small number of patients (20%). As such, the anti-EGFR is being tried even though low drug response is expected in most patients.

At present, expensive genetic testing such as RAS mutation and BRAF mutation is being conducted to investigate response to the anti-EGFR drugs. However, the test takes a long time and the rate of successful prediction is only about 50%. As such, due to the difference in the method of diagnosing tumors in a patient and the method of treating the tumors, effective treatment is not being made even after accurate diagnosis has been made. As a result, the rate of perfect cure is very low.

In order to solve these problems, many multinational pharmaceutical companies are trying to develop target therapeutic agents and companion diagnostic products recently. As a typical example, Herceptin, which is a targeted therapeutic agent used for the treatment of breast cancer, etc., has a market of 7 billion dollars a year. Companion diagnostic products such as HercepTest^{™} (DAKO), aimed at improving the treatment success rate of Herceptin, have increased the use of Herceptin greatly. As described above, there is a consistently increasing need for the development of a therapeutic agent with superior anticancer efficacy and a technology that allows fast and accurate prediction of therapeutic response at reasonable cost.

### [References of Related Art]

### [Patent Documents]

Patent document 1. Korean Patent Registration No. 10-1930399.

### [Disclosure]

### [Technical Problem]

The present disclosure is directed to providing a composition for preventing or treating colon cancer, which can specifically penetrate into cancer cells having anti-EGFR drug response.

The present disclosure is also directed to providing a composition and a method for predicting for predicting medicinal efficacy, which allow the prediction of drug response of the composition for preventing or treating colon cancer.

### [Technical Solution]

The present disclosure provides a composition for diagnosing colon cancer or predicting medicinal efficacy, which contains a complex wherein a fluorophore and a quencher are conjugated on both sides of a peptide represented by SEQ ID NO 1, which is cleaved by a lysosomal enzyme present in a tumor cell, and EGF (epidermal growth factor) is bound to the C-terminal of the peptide.

The fluorophore may be bound to the N-terminal of the peptide, and the quencher may be bound to the ε-amino group of a lysine or arginine amino acid residue of the peptide.

An anticancer drug may be further bound to the peptide.

The fluorophore may be any one selected from a group consisting of fluorescein, FITC (fluorescein isothiocyanate), Oregon green, Texas red, Cy2, Cy3, Cy3B, Cy3.5, Cy5, Cy5.5, Cy7, indocarbocyanine, rhodamine, oxacarbocyanine, thiacarbocyanine, merocyanine, pyridyloxazole, nitrobenzoxadiazole, benzoxadiazole, Nile red, Nile orange, acridine yellow, auramine, crystal violet and malachite green.

The quencher may be one or more selected from a group consisting of TAMRA (6-carboxytetramethyl-rhodamine), BHQ1 (black hole quencher 1), BHQ2 (black hole quencher 2), BHQ3 (black hole quencher 3), NFQ (nonfluorescent quencher), DABCYL, Eclipse, DDQ (deep dark quencher), blackberry quencher and Iowa black.

The colon cancer may be epidermal growth factor receptor (EGFR)-positive colon cancer.

The complex may penetrate into a colon cancer cell and emit fluorescence as it is cleaved by an enzyme existing in the lysosome of the colon cancer cell and quenching by the fluorophore and the quencher is resolved.

The present disclosure also provides a method for providing information for predicting response to an epidermal growth factor receptor (EGFR)-targeting drug, which includes a step of treating a sample isolated from a cancer patient with the composition for diagnosing or detecting colon cancer and measuring fluorescence intensity.

The subject may be determined as responding to the EGFR drug if the fluorescence intensity is detected.

The present disclosure also provides a method for providing information for verifying the therapeutic effect of an anticancer drug, which includes a step of treating a colon sample isolated from a cancer patient who is taking the anticancer drug with the composition for diagnosing or detecting colon cancer and measuring fluorescence intensity.

It may be determined that the anticancer drug has no therapeutic effect for the colon cancer patient if the fluorescence intensity is detected.

The present disclosure also provides a pharmaceutical composition for preventing or treating colon cancer, which contains:
a) a complex wherein a fluorophore and a quencher are conjugated on both sides of a peptide represented by SEQ ID NO 1, which is cleaved by a lysosomal enzyme present in a tumor cell, and EGF (epidermal growth factor) is bound to the C-terminal of the peptide; and
b) a prodrug complex wherein EGF (epidermal growth factor) and an anticancer drug are conjugated on both sides of a peptide represented by SEQ ID NO 1 or SEQ ID NO 2, which is cleaved by a lysosomal enzyme present in a tumor cell.

The colon cancer may be EGFR-positive colon cancer.

In the prodrug complex b), the peptide and the EGF may be covalently bonded by a crosslinking agent.

The crosslinking agent may be one or more selected from a group consisting of protein A, carbodiimide, N-succinimidyl S-acetylthioacetate (SATA), N-succinimidyl 3-(2-pyridyldithio)propionate (SPDP) and sulfosuccinimidyl 4-(N-maleimidomethyl)cyclohexane-1-carboxylate (Sulfo-SMCC).

The anticancer drug may be one or more selected from a group consisting of doxorubicin, cyclophosphamide, mechlorethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cytarabine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, ixabepilone, vinblastine, vincristine, vindesine, vinorelbine, estramustine, maytansine, DM1 (mertansine), DM4, dolastatin, auristatin E, auristatin F, monomethyl auristatin E, monomethyl auristatin F and derivatives thereof.

### [Advantageous Effects]

A complex of the present disclosure includes EGF, but is affected more by lysosomal activity rather than the EGFR signaling pathway, and thus can overcome the shortcomings of the existing EGF therapy-based diagnostic and therapeutic compositions and provide successfully personalized and improved effects of treating, preventing and alleviating cancer.

In particular, a diagnostic composition and a composition for predicting drug response according to the present disclosure enable the prediction of the anticancer performance of a therapeutic composition of the present disclosure in a subject in advance, and thus enables anticancer treatment to be designed stably.

### [Brief Description of Drawings]

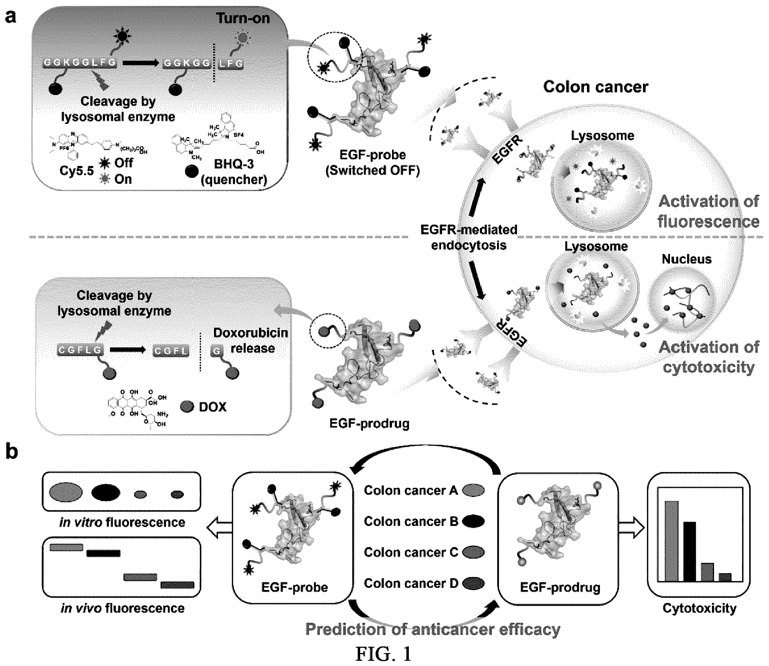

FIG. 1a schematically illustrates the structure and mechanism of a complex of the present disclosure for targeting cancer, and a composition for diagnosing cancer and a composition for treating cancer based thereon.
FIG. 1b schematically illustrates the structure and mechanism of a complex of the present disclosure for targeting cancer, and a composition for diagnosing cancer and a composition for treating cancer based thereon.
FIG. 2a schematically shows a process of synthesizing EGF-probe in Preparation Example 2.
FIG. 2b schematically shows a process of synthesizing EGF-prodrug in Preparation Example 5.
FIGS. 3a-3d show HPLC analysis results.

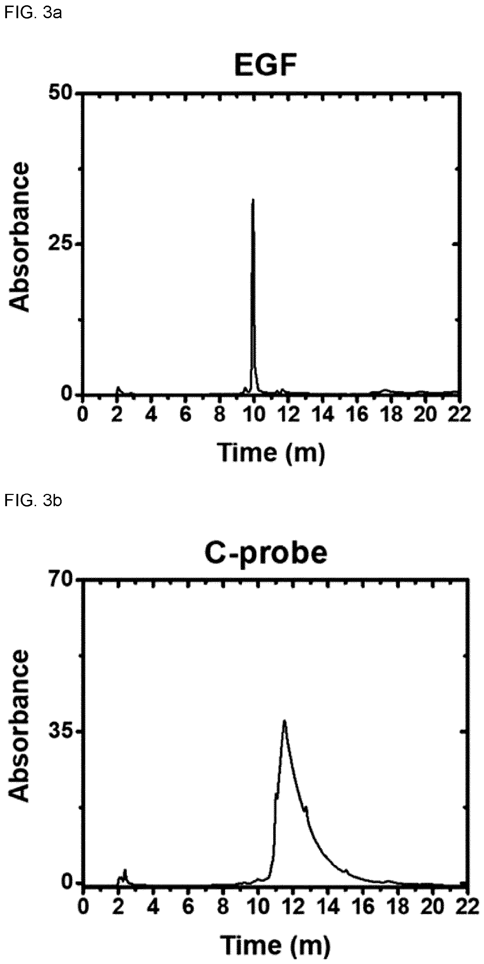

FIG. 3a shows an HPLC analysis result for EGF.
FIG. 3b shows an HPLC analysis result for C-probe of Preparation Example 2.

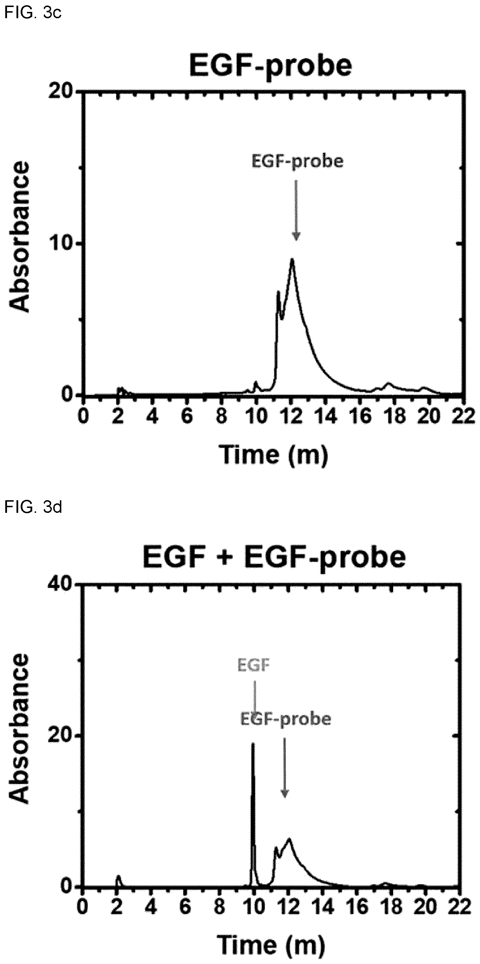

FIG. 3c shows an HPLC analysis result for EGF-probe of Preparation Example 3.
FIG. 3d shows an HPLC analysis result for a mixture of EGF and EGF-probe.

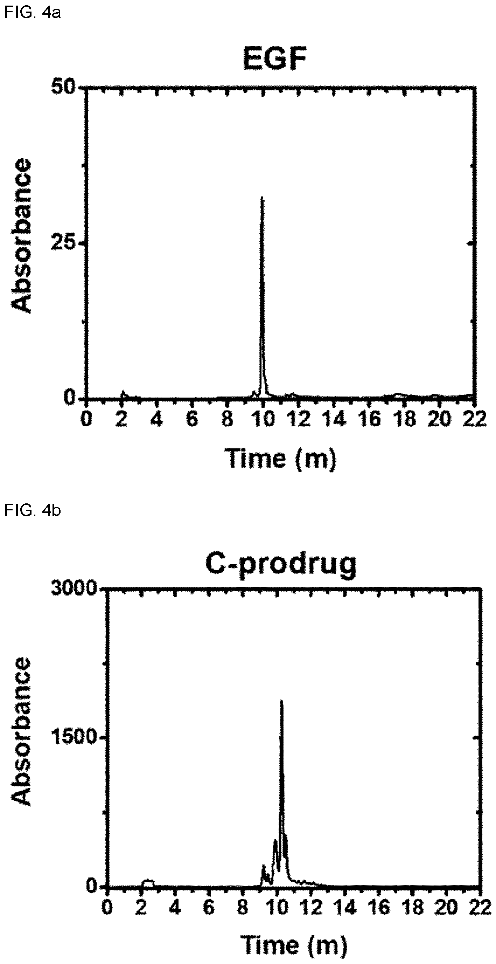

FIGS. 4a-4d show HPLC analysis results.
FIG. 4a shows an HPLC analysis result for EGF.
FIG. 4b shows an HPLC analysis result for C-prodrug of Preparation Example 4.

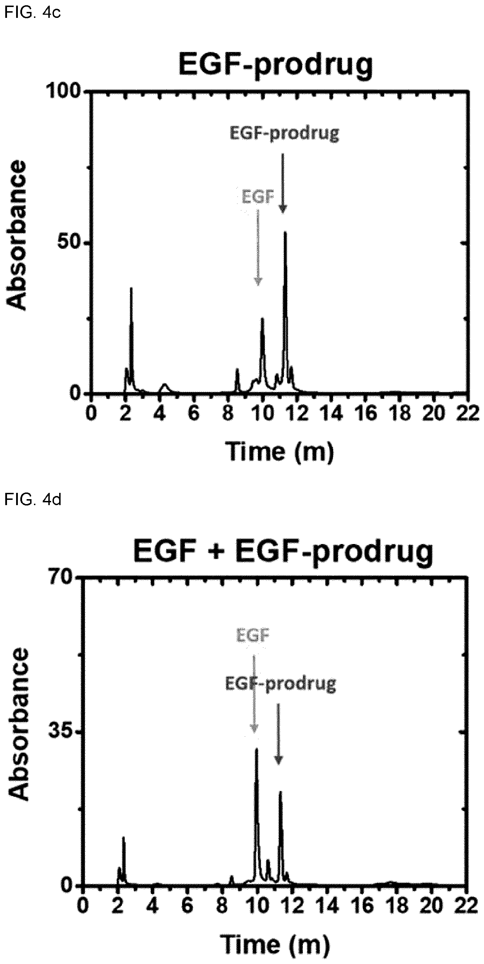

FIG. 4c shows an HPLC analysis result for EGF-prodrug of Preparation Example 5.
FIG. 4d shows an HPLC analysis result for a mixture of EGF and EGF-prodrug.

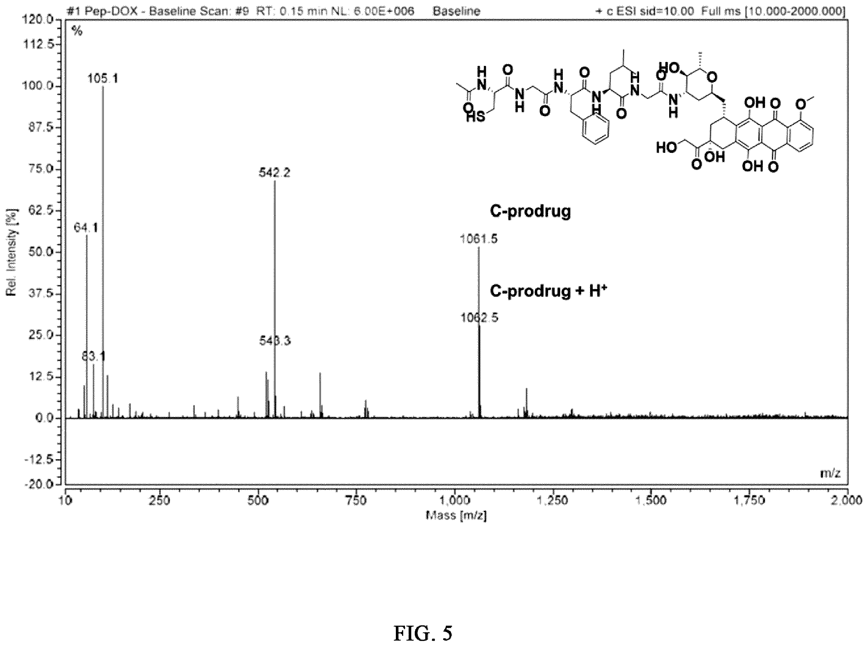

FIG. 5 shows a mass spectrum of C-prodrug.

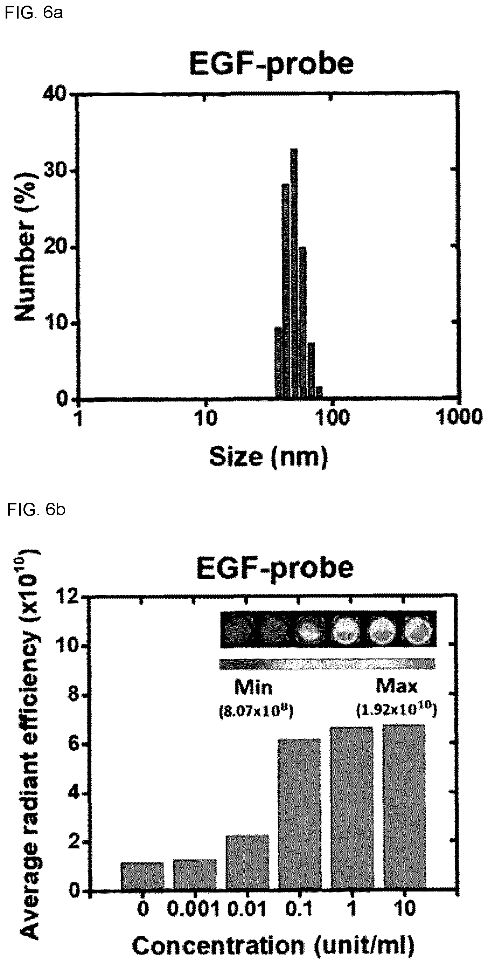

FIG. 6a shows the size distribution of EGF-probe.
FIG. 6b shows the fluorescence intensity of EGF-probe.

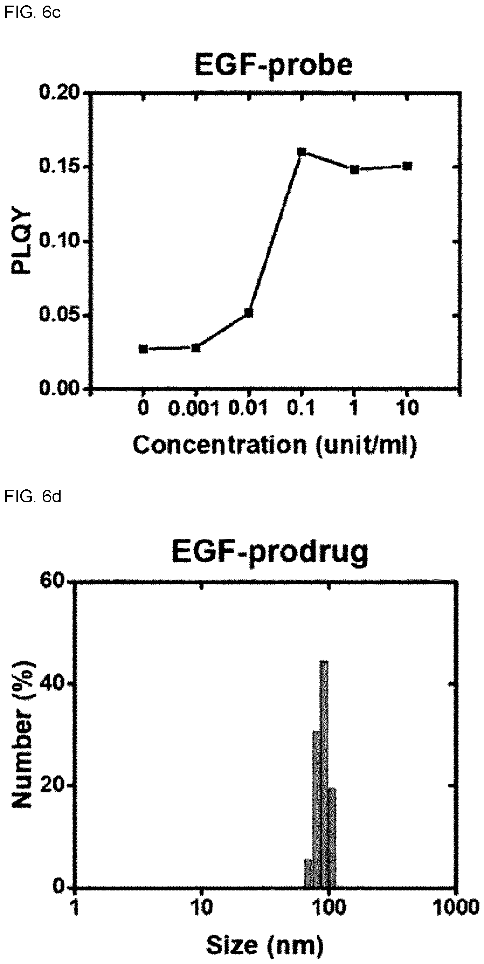

FIG. 6c shows the photoluminescence quantum yield (PLQY) of EGF-probe in the presence of papain of various concentrations.
FIG. 6d shows the size distribution of EGF-prodrug.

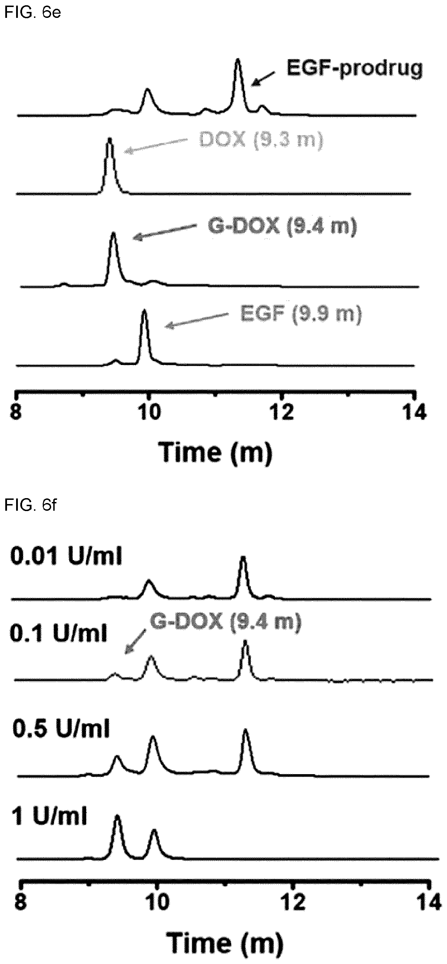

FIG. 6e shows the HPLC chromatograms of EGF-prodrug, DOX, G-DOX and EGF.
FIG. 6f shows the HPLC chromatograms of EGF-prodrug in the presence of papain at various concentrations.

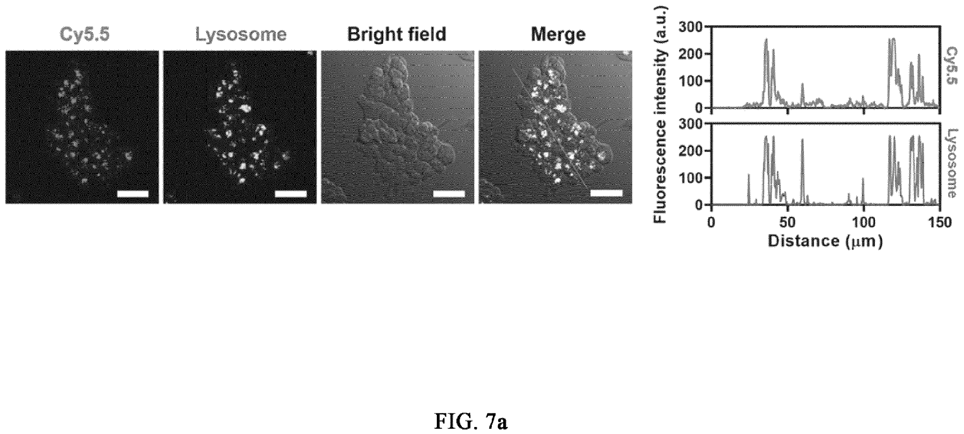

FIG. 7a shows fluorescence intensity (right) and fluorescence images (left) obtained by staining lysosomes with LysoTracker Green for observation of co-localization of Cy5.5 fluorescence (red) recovered from lysosomes (green) and EGF-probe in HCT-15 colorectal cancer cells treated with EGF-probe for 4 hours. Bar = 50 µm.

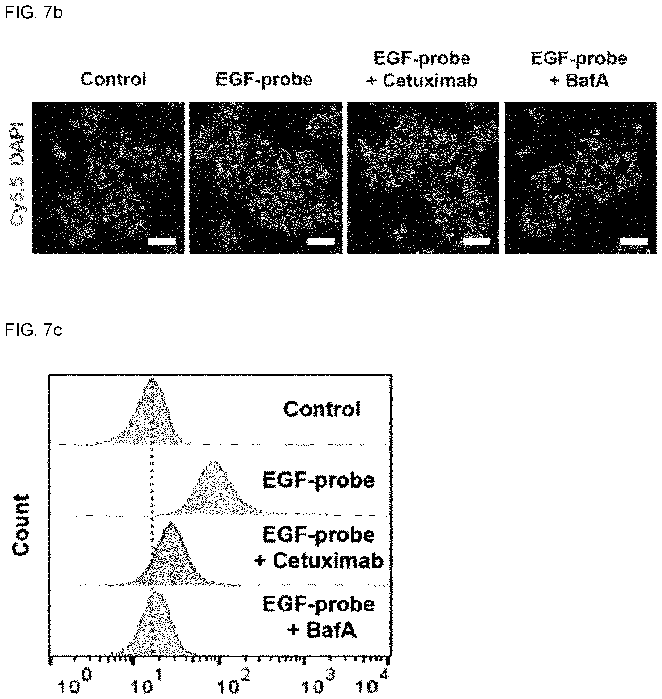

FIG. 7b shows confocal microscopic images of HCT-15 cells treated with EGF-probe and cetuximab (EGFR inhibitor) or EGF-probe and BafA (lysosome inhibitor).
FIG. 7c shows a flow cytometry analysis result of HCT-15 cells treated with EGF-probe and cetuximab (EGFR inhibitor) or EGF-probe and BafA (lysosome inhibitor) (DAPI: nuclei, bar = 50 µm).

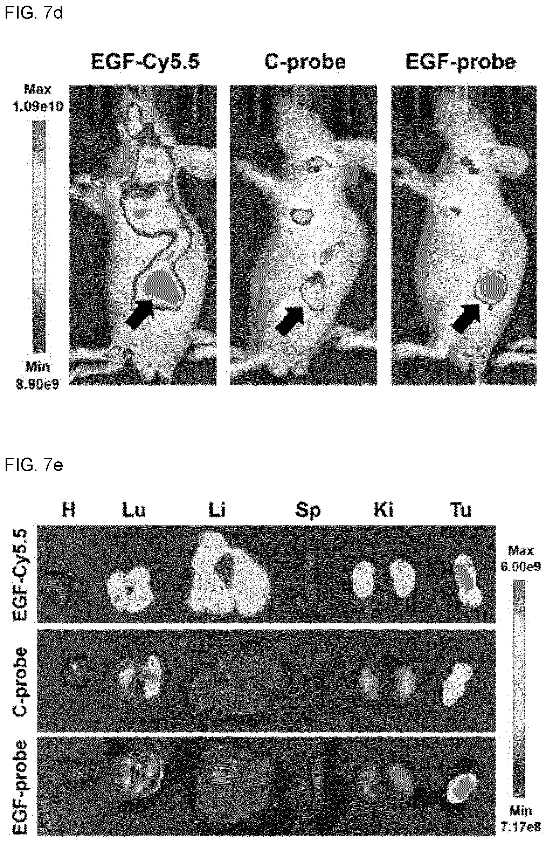

FIG. 7d shows IVIS images obtained 24 hours after intravenously administering EGF-Cy5.5, C-probe or EGF-probe to HCT-15-bearing mice.
FIG. 7e shows the fluorescence images of various organs obtained 24 hours after intravenously administering EGF-Cy5.5, C-probe or EGF-probe to HCT-15-bearing mice.

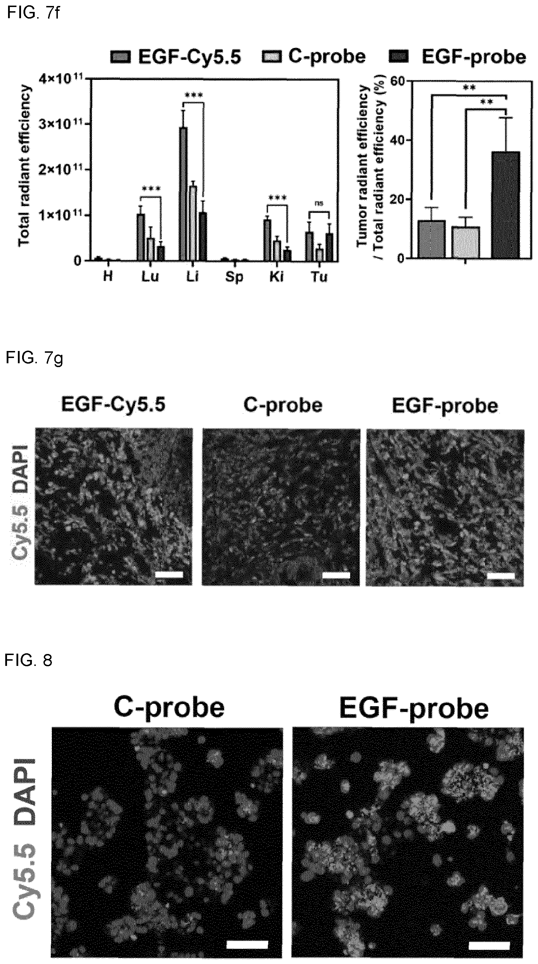

FIG. 7f shows the fluorescence intensity of various organs obtained from HCT-15-bearing mice treated with EGF-Cy5.5, C-probe or EGF-probe (left) and the fluorescence intensity of tumors divided by the fluorescence intensity of the total organs (right).
FIG. 7g shows the fluorescence images of tumor tissues obtained from mice to which GF-Cy5.5, C-probe or EGF-probe was administered. Bar = 50 µm.
FIG. 8 shows the confocal microscopic images of HCT-15 cells treated with C-probe or EGF-probe (10 µg/mL) for 2 hours.

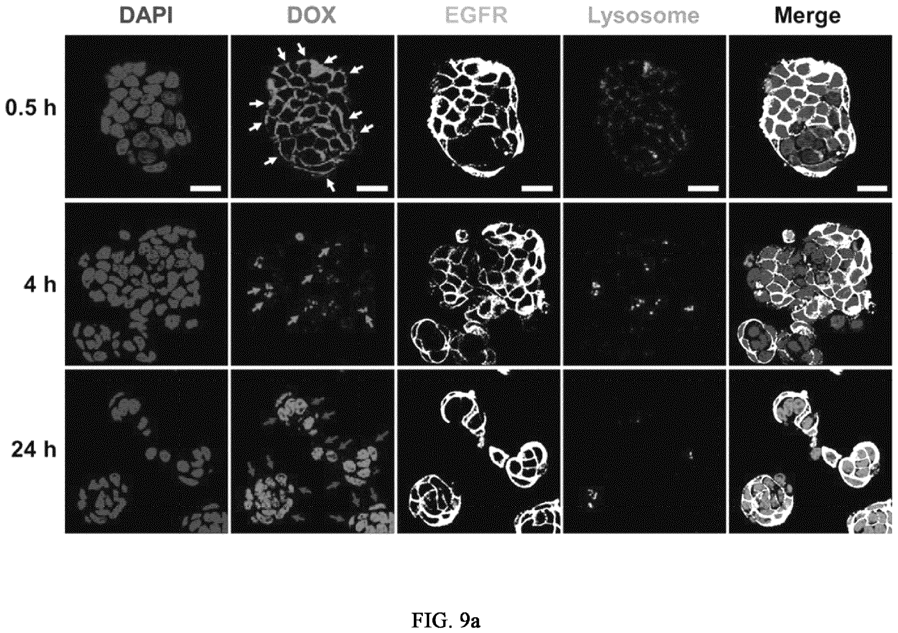

FIG. 9a shows the time-lapse images of DOX (red) in HCT-15 cells treated with EGF-prodrug.

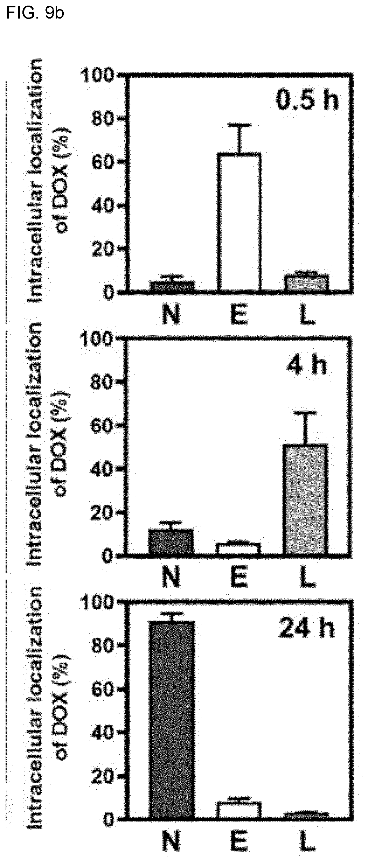

FIG. 9b shows a result of analyzing the intracellular localization of DOX at 0.5 hour, 4 hours and 24 hours after treatment with EGF-prodrug (N: nuclei, E: EGFRs, L: lysosomes). Bar = 20 µm.

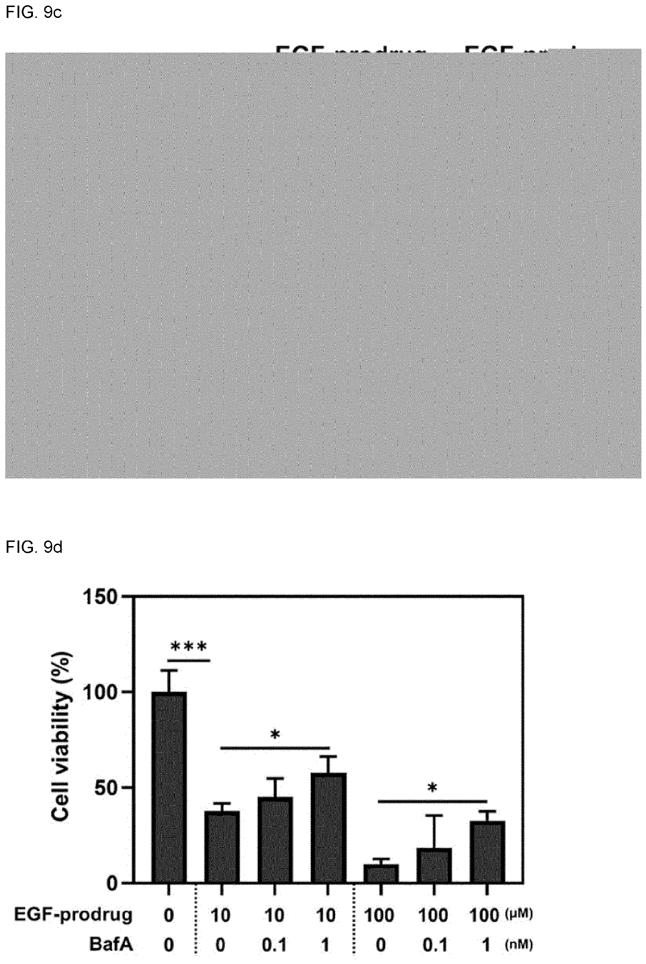

FIG. 9c shows the fluorescence microscopic images of HCT-15 cells obtained at 4 hours and 24 hours after treating with cetuximab or BafA and EGF-prodrug. Bar = 50 µm.
FIG. 9d shows a result of measuring cytotoxicity after treating HCT-15 cells with EGF-prodrug (0, 10 and 100 µM) or BafA (0, 0.1 and 1 nM) at various concentrations. n = 5, * P < 0.05, *** P < 0.001.

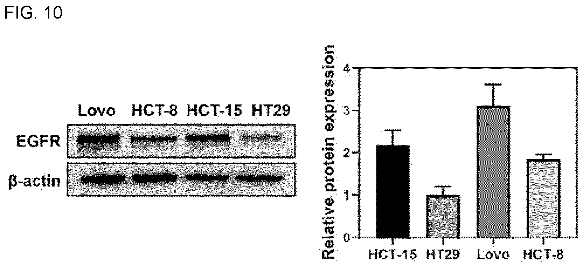

FIG. 10 shows a result of measuring EGFR expression level in HCT-15, HT29, Lovo and HCT-8 cells by western blot (n = 3).

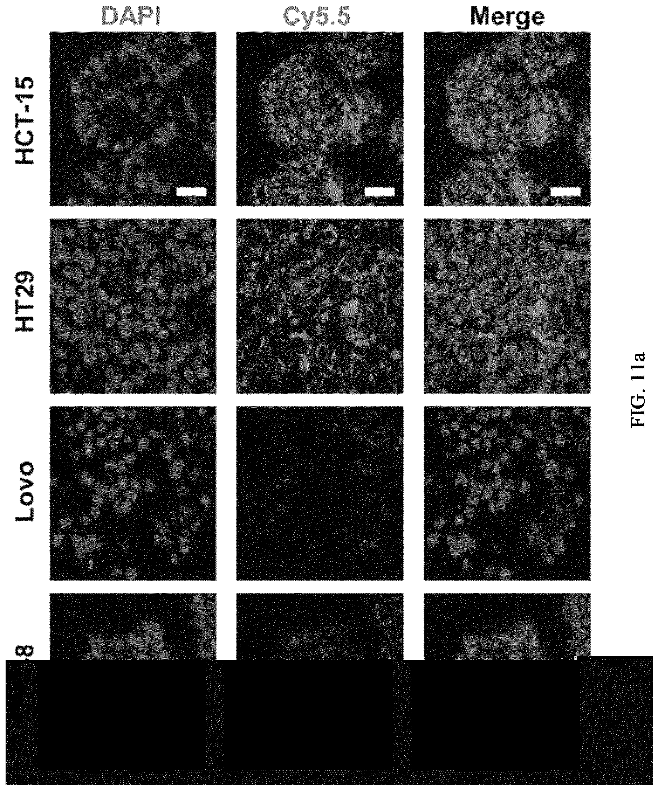

FIG. 11a shows fluorescence images obtained at 4 hours after treating HCT-15, HT29, Lovo and HCT-8 cells with EGF-probe. Bar = 50 µm.

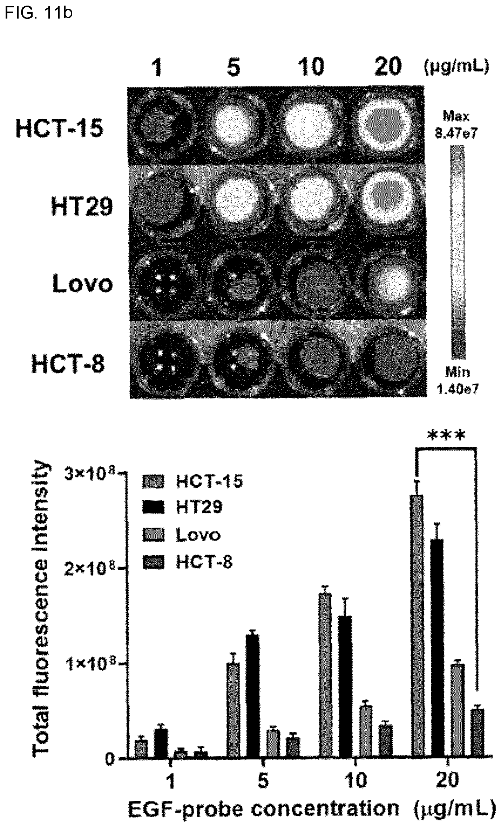

FIG. 11b shows the IVIS fluorescence images of cell lysates obtained by treating HCT-15, HT29, Lovo and HCT-8 cells with EGF-probe at various concentrations (top) and a result of quantifying fluorescence signals (bottom). n = 3, *** P < 0.001.

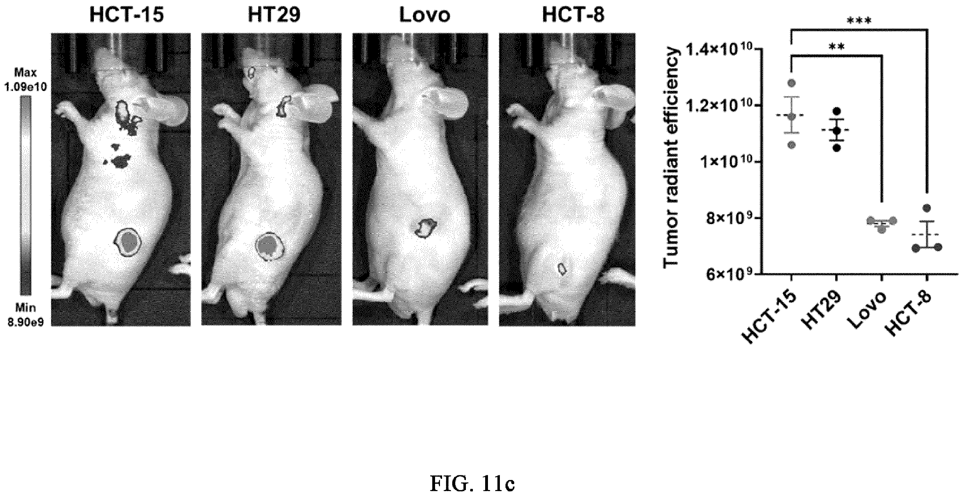

FIG. 11c shows the in-vivo IVIS images of mice in which HCT-15, HT29, Lovo and HCT-8 cells are transplanted obtained after administering EGF-probe (5 mg/kg).

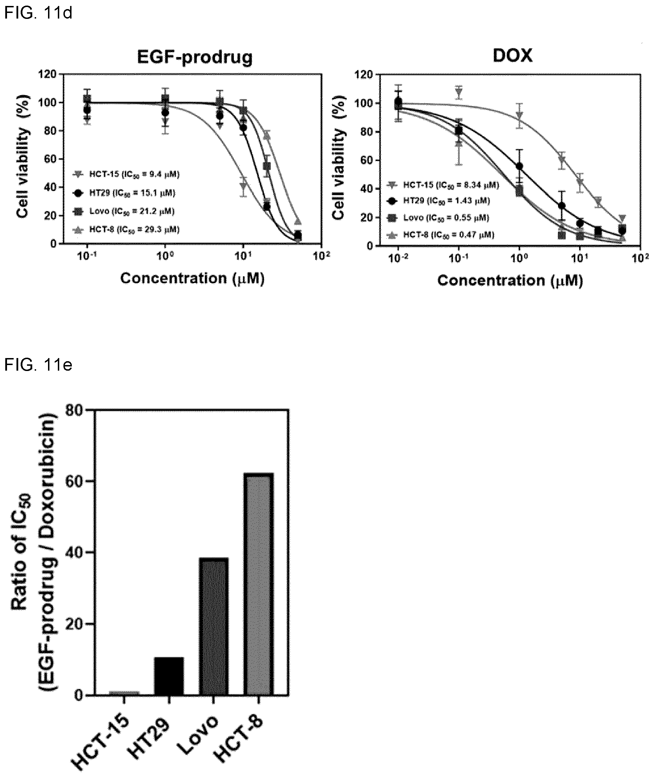

FIG. 11d shows a result of measuring cytotoxicity after treating HCT-15, HT29, Lovo and HCT-8 cells with EGF-prodrug and free DOX at various concentrations (n = 5).
FIG. 11e shows the IC₅₀ ratio of EGF-prodrug versus free DOX in HCT-15, HT29, Lovo and HCT-8 cells.

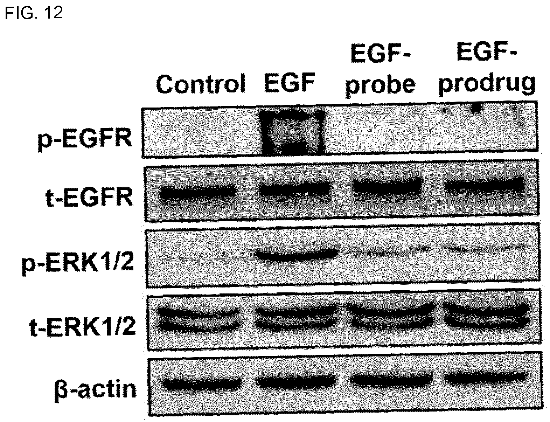

FIG. 12 shows a result of treating HCT-15 cells with PBS (control group), EGF, EGF-probe and EGF-prodrug (50 ng/mL of EGF) for 20 minutes and investigating phosphorylation of EGFR and ERK1/2.

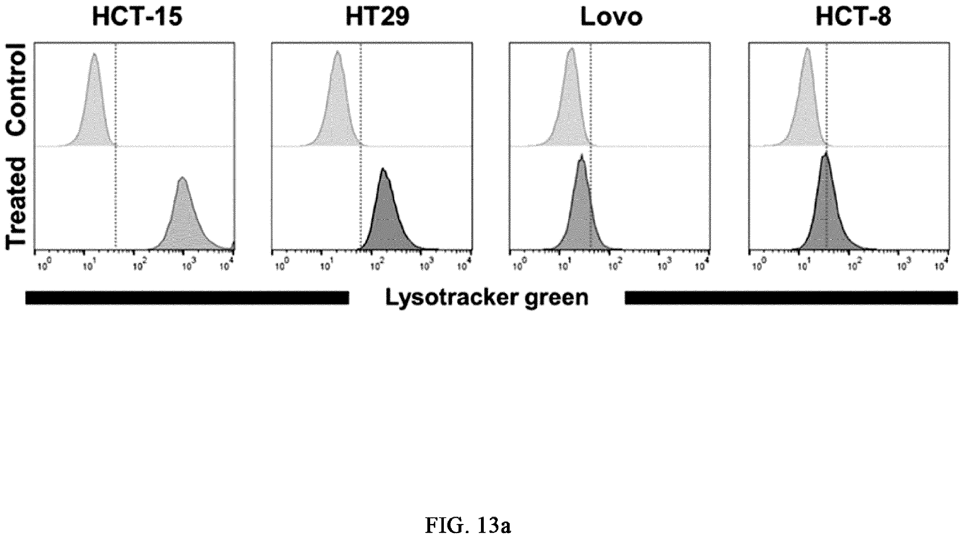

FIG. 13a shows a result of analyzing the intracellular expression level of EGFR and LAMP-1 in HCT-15, HT29, Lovo and HCT-8 cells by western blot.

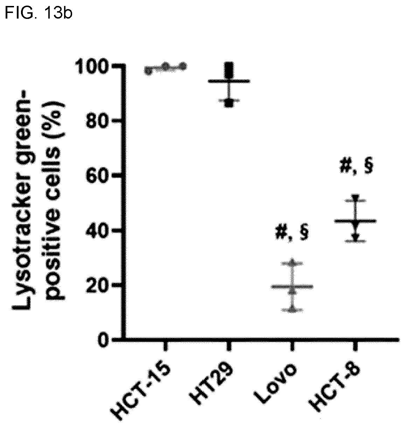

FIG. 13b shows a result of analyzing rectal cancer cells treated with LysoTracker Green by flow cytometry.

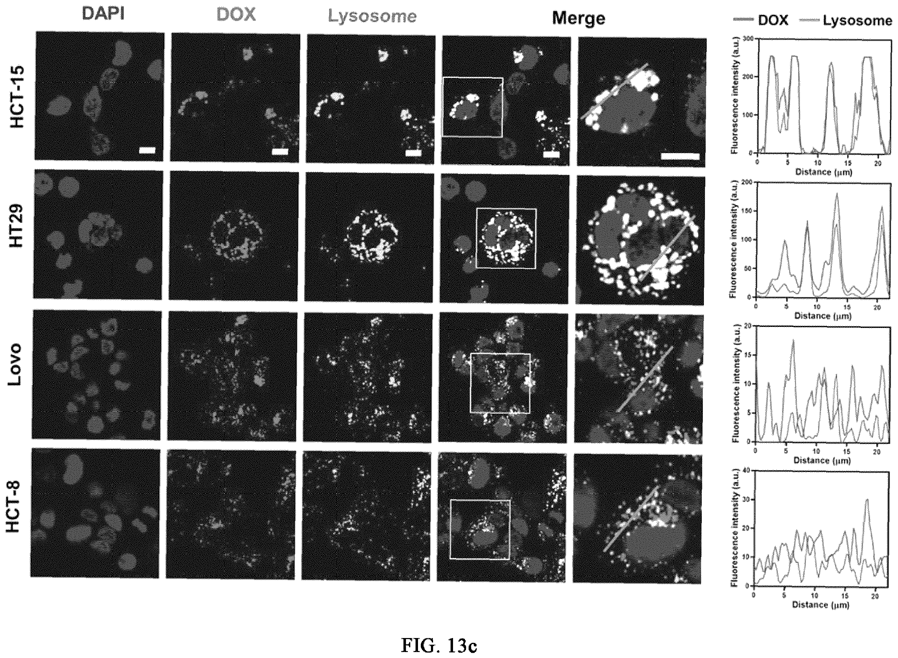

FIG. 13c shows confocal microscopic images obtained at 4 hours after treating HCT-15, HT29, Lovo and HCT-8 cells with EGF-prodrug.

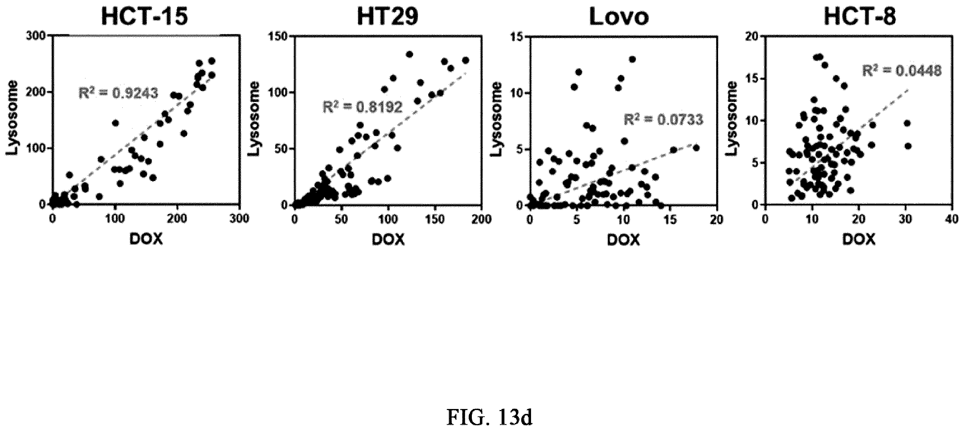

FIG. 13d shows a result of measuring fluorescence intensity at 4 hours after treating HCT-15, HT29, Lovo and HCT-8 cells with EGF-prodrug.

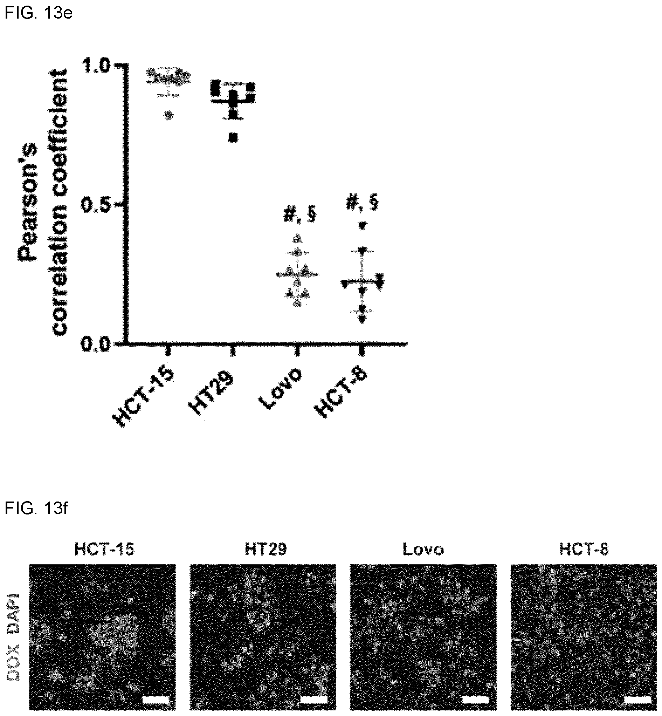

FIG. 13e shows the correlation between the fluorescence intensity of DOX and lysosomes.
FIG. 13f shows fluorescence images obtained at 24 hours after treating HCT-15, HT29, Lovo and HCT-8 cells with EGF-prodrug.

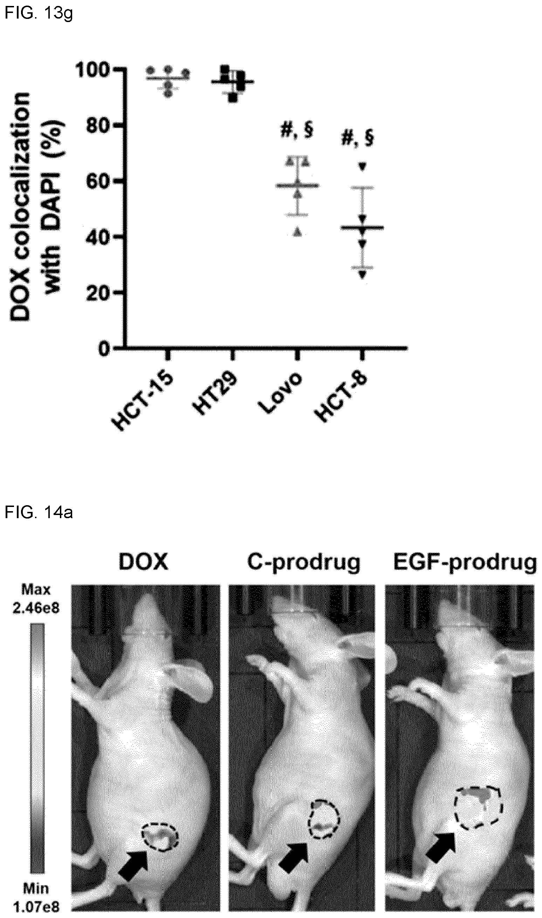

FIG. 13g shows a result of quantifying the ratio of co-localization of DAPI (cell nuclei) with DOX.
FIG. 14a shows the IVIS fluorescence images of HCT-15-bearing mice at 6 hours after administration of DOX (5 mg/kg), C-prodrug (5 mg/kg of DOX) or EGF-prodrug (5 mg/kg of DOX).

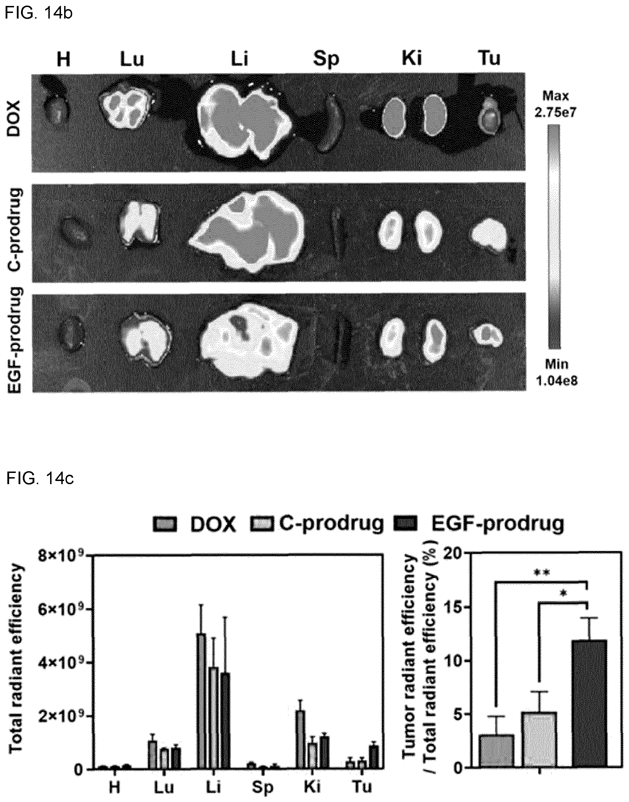

FIG. 14b shows fluorescence images showing the ex-vivo distribution of intravenously administered DOX, C-prodrug or EGF-prodrug (H: heart, Lu: lung, Li: liver, Sp: spleen, Ki: kidney, Tu: tumor).
FIG. 14c shows the fluorescence intensity of various organs (left) and the fluorescence intensity of tumors divided by the fluorescence intensity of the total organs (right).

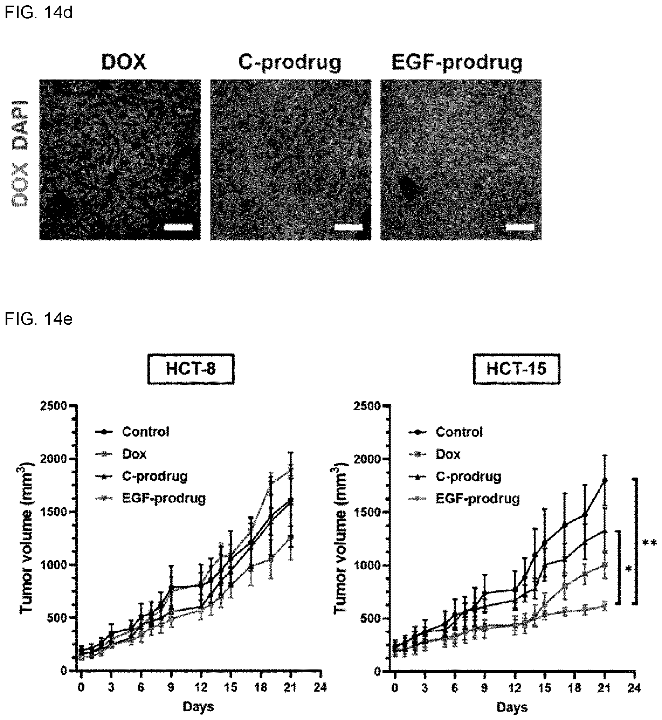

FIG. 14d shows the fluorescence images of tumor tissues isolated from mice to which DOX, C-prodrug or EGF-prodrug is administered. Bar = 50 µm.
FIG. 14e shows a result of injecting DOX, C-prodrug or EGF-prodrug (1 mg/kg of DOX, 6 times every 3 days) to HCT-8- or HCT-15-bearing mice and measuring tumor size for 21 days.

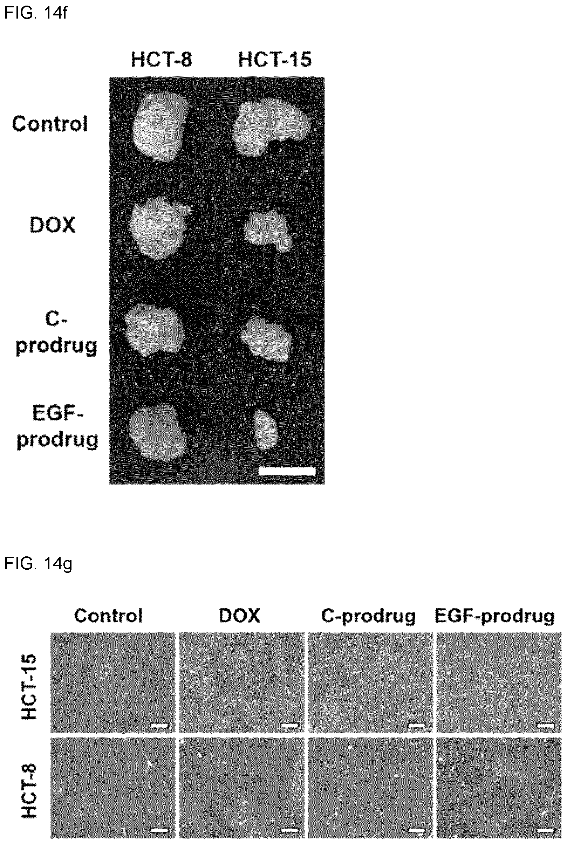

FIG. 14f shows tumor tissues taken from HCT-8- or HCT-15-bearing mice on day 24 after injection of PBS, DOX, C-prodrug or EGF-prodrug.
FIG. 14g shows a result of staining tumor tissues taken from HCT-8- or HCT-15-bearing mice on day 24 after injection of PBS, DOX, C-prodrug or EGF-prodrug with H&E.

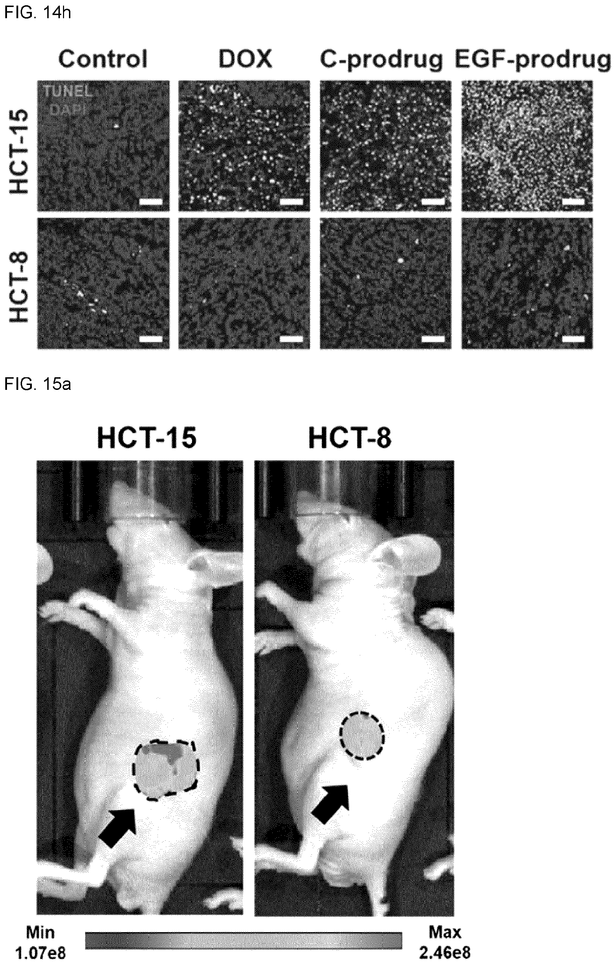

FIG. 14h shows a result of staining tumor tissues taken from HCT-8- or HCT-15-bearing mice on day 24 after injection of PBS, DOX, C-prodrug or EGF-prodrug with TUNEL. Bar = 1 cm.
FIG. 15a shows the IVIS fluorescence images of HCT-8- or HCT-15-bearing mice at 6 hours after treatment with EGF-prodrug (5 mg/kg of DOX).

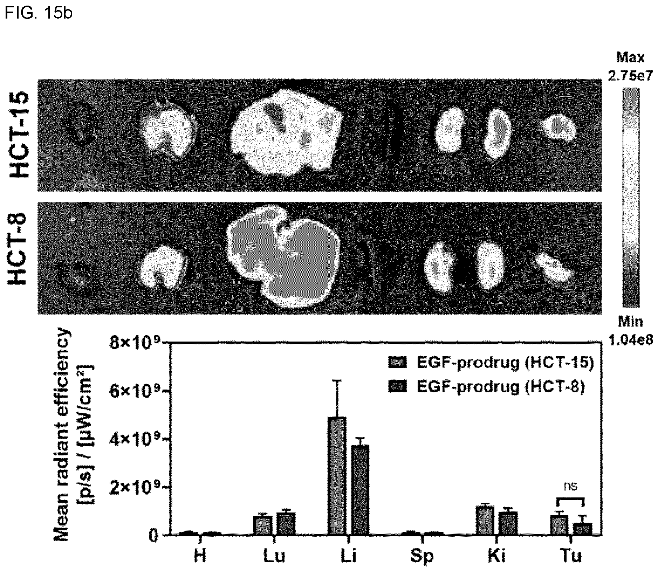

FIG. 15b shows a result of measuring the ex-vivo distribution and fluorescence intensity of EGF-prodrug.

### [Best Mode]

Hereinafter, the present disclosure is described in detail.

In the present specification, the term "peptide" refers to a linear molecule formed as amino acid residues are linked by peptide bonds.

Typical amino acids and their abbreviations are: alanine (Ala, A), isoleucine (Ile, I), leucine (Leu, L), methionine (Met, M), phenylalanine (Phe, F), proline (Pro, P), tryptophan (Trp, W), valine (Val, V), asparagine (Asn, N), cysteine (Cys, C), glutamine (Gln, Q), glycine (Gly, G), serine (Ser, S), threonine (Thr, T), tyrosine (Try, Y), aspartic acid (Asp, D), glutamic acid (Glu, E), arginine (Arg, R), histidine (His, H), lysine (Lys, K).

The present disclosure relates to a composition for diagnosing colon cancer, which contains a complex wherein a fluorophore and a quencher are conjugated on both sides of a peptide represented by SEQ ID NO 1, which is cleaved by a lysosomal enzyme present in a tumor cell, and EGF (epidermal growth factor) is bound to the C-terminal of the peptide.

The existing substances for targeting colon cancer cells have various problems such as fast degradation in vivo and difficulty in reaching the target site, or difficulty in penetrating into colon cancer cells. Anticancer strategies targeting EGFR have been reported recently. However, because EGFR-targeting drugs are not applicable to all cancers, they have to be used in combination with other anticancer strategies. In addition, the efficacy of the EGFR-targeting drugs varies greatly depending on individuals. Nevertheless, a platform for studying the anticancer efficacy of the various EGFR-targeting drugs is not available yet. In order to solve this problem, the inventors of the present disclosure have made efforts to invent a substance which can accurately target colon cancer cells based on EGFR by effectively penetrating into the cells and emitting fluorescence as it is degraded by lysosomes present in the colon cancer cells.

The composition for diagnosing or detecting colon cancer may penetrate into a colon cancer cell and emit fluorescence as it is cleaved by an enzyme existing in the lysosome of the colon cancer cell and quenching by the fluorophore and the quencher is resolved.

The complex of the present disclosure has a structure of: a peptide consisting of an amino acid sequence represented by SEQ ID NO 1; and EGF (epidermal growth factor) bound at the C-terminal of the peptide. Specifically, EGF may be bound at the C-terminal (carboxyl group) of the amino acid sequence represented by SEQ ID NO 1 through NHS-EDC reaction.

The EGF may be a natural EGF ligand. It has more advantages than EGFR-targeting antibodies. Specifically, it is a 6-kDa protein consisting of 53 residues. It can penetrate more deeply into a cancer cell due to high affinity and can be removed quickly from the body due to a small molecular weight.

The colon cancer targeted by the composition containing the complex may be an epidermal growth factor receptor (EGFR)-positive colon cancer.

According to an exemplary embodiment of the present disclosure, a fluorophore and a quencher may be conjugated on both sides of the peptide. In this case, the complex of the composition for diagnosing or detecting colon cancer is designed such that it is quenched normally and exhibits fluorescence intensity close to 0, but emits fluorescence when it is degraded by a lysosome present in a cancer cell. Therefore, when the protein-fluorophore complex penetrates into a cancer cell, it is effectively degraded by a lysosome present in the cancer cell and emits fluorescence for the cancer cell with high lysosomal activity.

That is to say, when the composition for diagnosing or detecting colon cancer is treated in vivo or ex vivo, the complex binds specifically to a colon cancer cell having EGFR, i.e., an epidermal growth factor receptor (EGFR)-positive colon cancer. Upon binding to EGFR, the complex is introduced into the cell and fluorescence is emitted as the peptide is cleaved and the fluorophore is separated from the quencher by an enzyme present in the lysosome of the cell. Therefore, fluorescence is not emitted in an EGFR-negative cancer cell. In addition, even when the complex is introduced into a cancer cell, the complex does not emit fluorescence if there is no lysosomal enzyme (cathepsin B) in the cancer cell because it exists stably without being cleaved (even if EGF is degraded by lysosomal degradation, etc. in the cell, no fluorescence is emitted because the fluorophore and the quencher are bound to the peptide).

Because the composition for diagnosing or detecting colon cancer has several safeguards as described above, it can accurately target a cancer cell and, at the same time, can predict the therapeutic effect of an EGFR-based anticancer drug.

If the complex has a structure of fluorophore-EGF-quencher without the peptide represented by SEQ ID NO 1, the therapeutic effect of an EGFR-based anticancer drug cannot be predicted accurately because the effect of lysosomal activity is not considered at all.

That is to say, the composition for diagnosing or detecting colon cancer of the present disclosure has excellent biocompatibility and few side effects and can specifically diagnose, detect or image cancer cells, particularly cancer cells having EGFR. In addition, it was confirmed through various test examples that the composition for diagnosing or detecting colon cancer of the present disclosure can be used effectively to predict the therapeutic effect of an EGFR-based anticancer drug.

In addition, since the composition for diagnosing or detecting colon cancer is easily dissolved in a physiological solution, it is absorbed easily and exhibits excellent bioavailability. Furthermore, it is very useful for diagnosing, detecting or imaging epidermal growth factor receptor (EGFR)-positive colon cancer cells because of significantly improved stability and cancer cell specificity.

The peptide may be composed of an amino acid sequence represented by SEQ ID NO 1. The peptide may be a GFLG-based peptide that can be cleaved by lysosomal degradative enzymes in a colon cancer cell, and may include a peptide in which one or more glycine, lysine (K) or arginine (R) amino acid residues are bound at the C-terminal.

The fluorophore may be bound at the N-terminal of the peptide, and the quencher may be bound at the ε-amino group (side chain amino group) of the lysine or arginine amino acid residue of the peptide.

Various well-known technologies may be used for this. Specifically, the quencher or the fluorophore may be bound to the peptide in DMSO in the presence of N-methylmorpholine (NMM) and 4-dimethylaminopyridine (DMAP) catalysts. The synthesis process consists of introduction of the fluorophore, removal of a protecting group and introduction of the quencher. Each step can be identified by high-performance liquid chromatography (HPLC). If the peptide has an amino group and the fluorophore and the quencher have N-hydroxysuccinimide (NHS) functional groups, covalent bonding may be present between the molecules. And, if the peptide has a COOH group and the fluorophore and the quencher have amino groups, a crosslinker (e.g., dicyclohexylcarbodiimide (DCC) or 1-ethyl-3-(3-dimethylaminopropyl)carbodiimide (EDAC) hydrochloride) may be used because direct chemical bonding between the two residues is impossible.

The fluorophore may be any one selected from a group consisting of fluorescein, FITC (fluorescein isothiocyanate), Oregon green, Texas red, Cy2, Cy3, Cy3B, Cy3.5, Cy5, Cy5.5, Cy7, indocarbocyanine, rhodamine, oxacarbocyanine, thiacarbocyanine, merocyanine, pyridyloxazole, nitrobenzoxadiazole, benzoxadiazole, Nile red, Nile orange, acridine yellow, auramine, crystal violet and malachite green, specifically Cy5.5.

The quencher may be one or more selected from a group consisting of TAMRA (6-carboxytetramethyl-rhodamine), BHQ1 (black hole quencher 1), BHQ2 (black hole quencher 2), BHQ3 (black hole quencher 3), NFQ (nonfluorescent quencher), DABCYL, Eclipse, DDQ (deep dark quencher), blackberry quencher and Iowa black, specifically BHQ-3.

The fluorophore and the quencher are not specially limited to the above-described examples. Any compound exhibiting fluorescence may be used as the fluorophore and any compound capable of quenching the fluorescence of the fluorophore may be used as the quencher, as long as they can bind to the peptide represented by SEQ ID NO 1.

The peptide and the EGF may be covalently bonded by a crosslinking agent. The crosslinking agent may be one or more selected from a group consisting of protein A, carbodiimide, N-succinimidyl S-acetylthioacetate (SATA), N-succinimidyl 3-(2-pyridyldithio)propionate (SPDP) and sulfosuccinimidyl 4-(N-maleimidomethyl)cyclohexane-1-carboxylate (Sulfo-SMCC).

The composition for diagnosing or detecting colon cancer reacts with EGFR overexpressed in cancer tissues, is introduced into cancer cells and emits fluorescence as it is degraded by lysosomal degradative enzymes in the cells. The fluorophore may be Cy5.5, which is a cyanine fluorophore emitting near infrared fluorescence (650-900 nm). As the quencher, BHQ3 that can maximize quenching effect by absorbing the fluorescence of the fluorophore may be used. A near infrared fluorophore is suitable for in-vivo fluorescence imaging of large-sized animals such as human because it can image epidermal growth factor receptor (EGFR)-positive colon cancer cells at higher depths better than fluorescent molecules emitting light in the visible range.

According to another exemplary embodiment of the present disclosure, an anticancer drug may be bound to the peptide.

The anticancer drug may be one or more selected from a group consisting of doxorubicin, cyclophosphamide, mechlorethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cytarabine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, ixabepilone, vinblastine, vincristine, vindesine, vinorelbine, estramustine, maytansine, DM1 (mertansine), DM4, dolastatin, auristatin E, auristatin F, monomethyl auristatin E, monomethyl auristatin F and derivatives thereof, specifically doxorubicin.

The anticancer drug may be bound to the peptide by a method well known in the art such as covalent bonding, crosslinking, etc. The peptide of the present disclosure may be chemically modified, if necessary, as long as its activity is not lost.

In addition, if necessary, the prepared complex may be purified or identified using various technologies well known to those skilled in the art.

The complex may have a molecular weight of 18-21 kDa. If the molecular weight exceeds the upper limit, cancer cells may not be fluorescence-labeled effectively due to aggregation. And, if the molecular weight is below the lower limit, in-vivo imaging may be difficult due to decreased fluorescence intensity.

The diagnosis may include companion diagnosis which refers to diagnosis designed to provide information that is essential for the administration of a corresponding therapeutic agent.

Since the composition can be administered directly into the body, it may further contain a pharmaceutically acceptable carrier. The carrier includes carbohydrate-based compounds (e.g., lactose, amylose, dextrose, sucrose, sorbitol, mannitol, starch, cellulose, etc.), acacia gum, calcium phosphate, alginate, gelatin, calcium silicate, microcrystalline cellulose, polyvinylpyrrolidone, cellulose, water, syrup, salt solution, alcohol, Arabic gum, vegetable oil (e.g., corn oil, cottonseed oil, soybean oil, olive oil or coconut oil), polyethylene glycol, methyl cellulose, methyl hydroxybenzoate, propyl hydroxybenzoate, talc, magnesium stearate, mineral oil, etc., although not being limited thereto. The pharmaceutical composition of the present disclosure may further contain a lubricant, a wetting agent, a sweetener, a flavorant, an emulsifier, a suspending agent, a preservative, etc. in addition to the above-described ingredients. Suitable pharmaceutically acceptable carriers and preparations are described in detail in Remington's Pharmaceutical Sciences (19th ed., 1995).

The composition may be administered orally or parenterally. The parenteral administration may be made by one or more administration route selected from a group consisting of intravenous injection, subcutaneous injection and intramuscular injection.

The appropriate administration dosage of the composition varies depending on such factors as formulation method, administration method, the age, body weight, sex, pathological condition and diet of a patient, administration time, administration route, excretion rate and response sensitivity, and an ordinarily skilled physician can easily determine and prescribe an administration dosage effective for desired treatment or prevention. According to a specific exemplary embodiment of the present disclosure, the composition may be administered at a dosage of 0.0001-100 mg/kg (body weight). The administration may be made once or several times a day.

Another aspect of the present disclosure relates to a composition for identifying prognosis for therapeutic effect in a patient with EGFR-positive colon cancer, which contains a complex wherein a fluorophore and a quencher are conjugated on both sides of a peptide represented by SEQ ID NO 1, which is cleaved by a lysosomal enzyme present in a tumor cell, and EGF (epidermal growth factor) is bound to the C-terminal of the peptide.

The composition may be used to identify anticancer therapeutic effect or predict response to a specific drug in advance by treating the same to a colon cancer patient, particularly a patient with EGFR-positive colon cancer, administered with an anticancer drug and monitoring the presence of tumors. This type of diagnosis is also called companion diagnosis and is recognized as a very important field in relation to personalized medicine.

After administering the composition, if the presence or increase of a fluorescence signal is observed, it may be determined that the therapeutic effect of the anticancer drug is negative. And, if the fluorescence signal is decreased or not observed, it may be determined that the therapeutic effect of the anticancer drug is positive.

Another aspect of the present disclosure relates to a method for providing information for predicting response to an epidermal growth factor receptor (EGFR) drug, which includes a step of treating a sample isolated from a cancer patient with the composition for diagnosing or detecting colon cancer and measuring fluorescence intensity.

In the present disclosure, the term "patient" includes human or non-human animals. The term "non-human animal" may refer to a vertebrate, e.g., non-human primates, sheep, dog or rodents, e.g., mouse, rat or guinea pig. Specifically, the patient may be human. In the present specification, the term "patient" may be used interchangeably with a "subject" and an "individual".

The sample isolated from a cancer patient may be one or more selected from a group consisting of a tissue, a cell, whole blood, blood, saliva, sputum, cerebrospinal fluid and urine, although not being specially limited thereto.

If the fluorescence intensity is detected, the response of the subject to an epidermal growth factor receptor (EGFR)-targeting drug is increased significantly. Otherwise, if the fluorescence intensity is not detected, the drug response is decreased significantly. Therefore, if fluorescence is emitted when a subject or a sample of the subject is treated with the composition for diagnosing or detecting colon cancer, it may be predicted that the epidermal growth factor receptor (EGFR)-targeting drug will show efficacy in the subject. Accordingly, the composition may be used to provide a personalized anticancer drug by predicting drug response.

If the cancer patient has EGFR-positive colon cancer, important information for prescribing an epidermal growth factor receptor (EGFR)-targeting drug may be provided.

In the present disclosure, the 'epidermal growth factor receptor (EGFR)-targeting drug' is not specially limited as long as it is an anticancer drug which inhibits the activation of EGFR by binding thereto. Specifically, it may be one or more selected from a group consisting of cetuximab, bevacizumab, panitumumab and trastuzumab.

Another aspect of the present disclosure relates to a method for providing information for verifying the therapeutic effect of an anticancer drug, which includes a step of treating a colon sample isolated from a cancer patient who is taking the anticancer drug with the composition for diagnosing or detecting colon cancer.

In the present disclosure, the term "patient" includes human or non-human animals. The term "non-human animal" may refer to a vertebrate, e.g., non-human primates, sheep, dog or rodents, e.g., mouse, rat or guinea pig. Specifically, the patient may be human. In the present specification, the term "patient" may be used interchangeably with a "subject" and an "individual".

The sample isolated from a cancer patient may be one or more selected from a group consisting of a tissue, a cell, whole blood, blood, saliva, sputum, cerebrospinal fluid and urine, although not being specially limited thereto.

The colon cancer patient may be a patient with epidermal growth factor receptor (EGFR)-positive colon cancer.

If the fluorescence intensity is detected, it is determined that the anticancer drug has no therapeutic effect for the colon cancer patient. And, if the fluorescence intensity is undetected or decreased, the tumor size is decreased due to the therapeutic effect of the anticancer drug. Therefore, important information for determining the prognosis or therapeutic effect of the anticancer drug may be provided.

In the present disclosure, the 'anticancer drug' may be one or more selected from a group consisting of doxorubicin, cyclophosphamide, mechlorethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cytarabine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, ixabepilone, vinblastine, vincristine, vindesine, vinorelbine, estramustine, maytansine, DM1 (mertansine), DM4, dolastatin, auristatin E, auristatin F, monomethyl auristatin E, monomethyl auristatin F and derivatives thereof.

Another aspect of the present disclosure relates to a pharmaceutical composition for preventing or treating colon cancer, which contains:
a) a complex wherein a fluorophore and a quencher are conjugated on both sides of a peptide represented by SEQ ID NO 1, which is cleaved by a lysosomal enzyme present in a tumor cell, and EGF (epidermal growth factor) is bound to the C-terminal of the peptide; and
b) a prodrug complex wherein EGF (epidermal growth factor) and an anticancer drug are conjugated on both sides of a peptide represented by SEQ ID NO 1 or SEQ ID NO 2, which is cleaved by a lysosomal enzyme present in a tumor cell.

A detailed description of the complex a) will be omitted to avoid redundancy.

The prodrug complex b) may penetrate into a colon cancer cell and exhibit anticancer effect by releasing an anticancer drug as it is cleaved by an enzyme present in the lysosome.

In the prodrug complex b), EGF (epidermal growth factor) and an anticancer drug are conjugated on both sides of a peptide represented by SEQ ID NO 1 or SEQ ID NO 2.

In the prodrug complex b), the peptide and the EGF may be covalently bonded by a crosslinking agent. The crosslinking agent may be one or more selected from a group consisting of protein A, carbodiimide, N-succinimidyl S-acetylthioacetate (SATA), N-succinimidyl 3-(2-pyridyldithio)propionate (SPDP) and sulfosuccinimidyl 4-(N-maleimidomethyl)cyclohexane-1-carboxylate (Sulfo-SMCC). Specifically, EGF may be bonded to a thiol functional group at the C-terminal of the peptide by an SMCC linker.

The anticancer drug may be one or more selected from a group consisting of doxorubicin, cyclophosphamide, mechlorethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cytarabine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, ixabepilone, vinblastine, vincristine, vindesine, vinorelbine, estramustine, maytansine, DM1 (mertansine), DM4, dolastatin, auristatin E, auristatin F, monomethyl auristatin E, monomethyl auristatin F and derivatives thereof.

The composition of the present disclosure can be prescribed effectively to a colon cancer patient who is expected to show response for an EGFR-targeting drug as the complex a) and the prodrug complex b) are treated sequentially or simultaneously and the medicinal effect can be monitored in real time through fluorescence signals.

The pharmaceutical composition is very safe in that it exhibits toxicity specifically only for colon cancer cells, particularly colon cancer cells having EGFR, and exhibits no cytotoxicity for normal tissues.

In addition, synergistic effect may be achieved because the efficacy of the prodrug complex b) may be predicted in advance or at the same time by administering the complex a).

That is to say, when the pharmaceutical composition is treated in vivo or ex vivo, it binds specifically to a cancer cell having EGFR. Upon binding to the EGFR, the pharmaceutical composition is introduced into the cell and, as the peptide is cleaved by an enzyme present in the lysosome of the cell, the fluorophore and the quencher are separated from the complex a) and fluorescence and anticancer activity are exhibited as the anticancer drug is separated from the prodrug complex b). Accordingly, even when a cancer cell is present, a fluorescence signal is not observed and anticancer effect is not exhibited if it is EGFR-negative. In addition, even when the pharmaceutical composition is introduced into a cancer cell, cytotoxicity (anticancer effect) is not exhibited if there is no lysosomal enzyme (cathepsin B) in the cancer cell because the anticancer drug remains stable without being separated from the pharmaceutical composition (even if the EGF is degraded by the lysosome of the cell, cytotoxicity is not exhibited because the anticancer drug remains bound to the peptide).

Because the pharmaceutical composition has several safeguards as described above, it can accurately target a colon cancer cell and, at the same time, can predict the therapeutic effect of an EGFR-based anticancer drug.

In addition, the pharmaceutical composition is advantageous in that an accurate dosage may be administered because it exists as spherical particles in solution through self-assembly, which are stable in vivo and have a uniform size.

The term pharmaceutically effective amount encompasses "therapeutically effective amount" and "prophylactically effective amount". The term "therapeutically effective amount" refers to an amount of a drug or a therapeutic agent which, when used either alone or in combination with another therapeutic agent, can reduce the severity of the symptoms of a disease, increase the duration or frequency of a period with no symptoms of a disease, or prevent damage or disorder caused by a disease. The term "prophylactically effective amount" refers to an amount of a drug which reduces the occurrence or relapse of colon cancer in a subject having the risk of occurrence or relapse of cancer. The level of the effective amount may be determined in consideration of the factors including the severity of a disease, the age and sex of the subject, the activity of a drug, the sensitivity for the drug, administration time, administration route, excretion rate, treatment period and co-administered drugs and other factors well known in the medical field.

The term "administration" used in the present disclosure refers to physical introduction of a composition into a subject using any of various methods and delivery systems known to those having ordinary knowledge in the art. The administration route for the pharmaceutical composition of the present disclosure includes all administration routes. Specifically, the administration route includes parenteral administration, e.g., intravenous, intramuscular, subcutaneous, intraperitoneal, intraspinal or other parenteral administration routes such as administration by injection or infusion. For example, the composition of the present disclosure may be administered at once, several times and/or over one or more extended periods.

The administration dosage of the pharmaceutical composition of the present disclosure may vary depending on the age, sex and body weight of a patient. Specifically, 0.0001-100 mg/kg (body weight) of the composition of the present disclosure may be administered once or several times a day depending on the severity of the disease of a subject having cancer. In addition, the administration dosage may be increased or decreased depending on administration route, the severity of a disease, the sex, body weight and age of a patient, particularly the severity of cancer of the patient, etc.

In addition, the composition of the present disclosure may be administered as an individual therapeutic agent or in combination with another therapeutic agent. When it is administered in combination with another therapeutic agent, the composition of the present disclosure may be administered either sequentially or simultaneously with the another therapeutic agent. The another therapeutic agent may be may be a compound, a protein, etc. which facilitates cancer regression or inhibits tumor growth, and also includes other anticancer therapies such as radiation therapy.

In addition, the present disclosure may provide a method for treating or preventing colon cancer, which includes a step of administering the pharmaceutical composition for preventing or treating colon cancer to a colon cancer patient who has been predicted to exhibit response to an EGFR-targeting drug using the composition for diagnosing or detecting colon cancer.

The terms used in the method for treating or preventing colon cancer according to the present disclosure have the same meanings as those used in the pharmaceutical composition for treating or preventing colon cancer unless specially stated otherwise.

The treatment of colon cancer may refer to inhibition of tumor growth, for example, by about 10% or more, about 20% or more, about 40% or more, about 60% or more, or about 80% or more, as compared to an untreated subject.

### [Mode for Invention]

Hereinafter, the present disclosure will be described in more detail through examples, etc. However, the scope and contents of the present disclosure cannot be interpreted as being reduced or limited by the examples, etc. In addition, it is obvious that those having ordinary knowledge can easily carry out the present disclosure for the matters for which experimental results are not presented specifically based on the disclosure of the present disclosure including the examples, etc. and such changes and modifications are encompassed in the appended claims.

The experimental results presented below are only representative experimental results of examples and comparative examples.

### Experimental materials

DMSO (dimethyl sulfoxide), DMF(dimethylformamide), DOX (doxorubicin hydrochloride), DIPEA (N,N-diisopropylethylamine), NHS (N-hydroxysuccinimide), EDC (1-ethyl-3-(3-dimethylaminopropyl)carbodiimide), papain (derived from papaya latex), EGF (human epidermal growth factor), Sulfo-SMCC (sulfosuccinimidyl-trans-4-(N-maleimidomethyl)cyclohexane-1-carboxylate), DMAP (4-(dimethylamino)pyridine), NMM (4-methylmorpholine), anisole, trifluoroacetic acid, bafilomycin A1 (A1) and disposable PD 10 desalting columns were purchased from Sigma-Aldrich (MO, USA). Cyanine5.5 NHS ester, anti-EGFR antibody and anti-β-actin antibody were purchased from Abcam (Cambridge, UK). BHQ-3-succinimidly ester was purchased from Biosearch Technologies Inc. (Hoddesdon, UK). NH₂-Gly-Phe-Leu-Gly-Gly-Lys (Boc)-Gly-Gly-COOH(NH₂-GFLGGK(Boc)GG-COOH) and (Ac)Cys-Gly-Phe-Leu-Gly((Ac) CGFLG) peptide sequences were synthesized by Peptron Co. (Daejeon, Republic of Korea) and BeadTech Inc. (Gyeonggi-do, Republic of Korea). LysoTracker Green DND-26 was purchased from Invitrogen (CA, USA). A DeadEnd^{™} fluorescence TUNEL staining kit was purchased from Promega (WI, USA). Anti-phospho-EGFR antibody, anti-ERK1/2 antibody, anti-phospho-ERK1/2 antibody and HRP-linked horse anti-mouse secondary antibody were purchased from Cell Signaling Technology (MA, USA). HRP-linked goat anti-rabbit secondary antibody was purchased from Agilent Technologies (CA, USA).

### Statistical analysis

Quantitative in-vitro and in-vivo data were represented as mean ± standard deviation (SD). The tumor size depending on time was represented as mean ± standard error of measurement (SEM). P-value was calculated by one-way or two-way analysis of variance using the GraphPad Prism 8 software. P < 0.05 was regarded as statistically significant.

### Preparation Example 1. Synthesis of EGF-Cy5.5

EGF-Cy5.5 was synthesized by mixing human EGF and Cy5.5-NHS ester in 1 mL of DMSO, adding DMAP and NMM, and then stirring the reaction mixture at 37 °C for 2 hours. The EGF-Cy5.5 was purified using a disposable PD10 desalting column and stored in PBS (phosphate-buffered saline) at 4 °C prior to use.

### Preparation Example 2. Synthesis of C-probe

For synthesis of Cy5.5-GFLGGK(BHQ-3)GG-COOH, which is a control probe (C-probe), NH₂-GFLGGK(Boc)GG-COOH and Cy5.5-BHS ester were dissolved in 2 mL of DMSO. After adding NMM and DMAP, the reaction mixture was stirred at 37 °C for 2 hours. The reaction mixture was purified by analytical reversed-phase HPLC (1200 Infinity Series, Agilent Technologies) equipped with a C18 column. A blue solid product, Cy5.5-GELGGK(Boc)GG-COOH, was obtained by freeze-drying the reaction mixture. After dissolving in a mixed solvent (TFA (trifluoroacetic acid): distilled water: anisole = 95: 2.5: 2.5), the mixture was purified by analytical reversed-phase HPLC (1200 Infinity Series, Agilent Technologies) equipped with a C18 column. Cy5.5-GFLGGKGG-COOH was obtained as a result.

Subsequently, the Cy5.5-GFLGGKGG-COOH was mixed with BHQ-3-succinimidly ester and stirred at 37 °C for 2 hours. C-probe was synthesized by purifying the mixture by reversed-phase HPLC.

### Preparation Example 2. Synthesis of EGF-probe

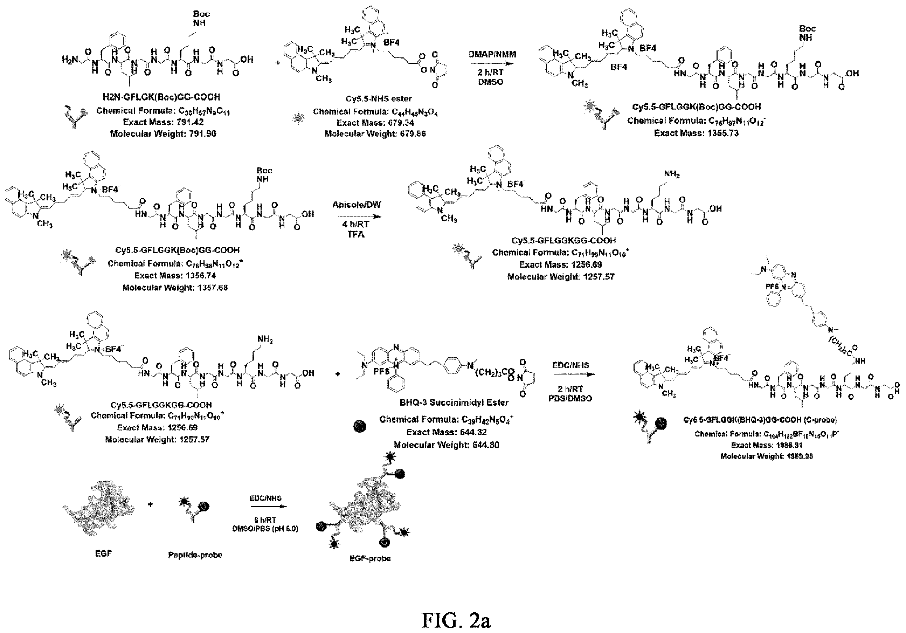

EGF-probe was synthesized according to FIG. 2a. NH₂-GFLGGK(Boc)GG-COOH and Cy5.5-BHS ester were dissolved in 2 mL of DMSO. After adding NMM and DMAP, the reaction mixture was stirred at 37 °C for 2 hours. The reaction mixture was purified by analytical reversed-phase HPLC (1200 Infinity Series, Agilent Technologies) equipped with a C18 column. A blue solid product, Cy5.5-GELGGK(Boc)GG-COOH, was obtained by freeze-drying the reaction mixture. After dissolving in a mixed solvent (TFA (trifluoroacetic acid): distilled water: anisole = 95: 2.5: 2.5), the mixture was purified by analytical reversed-phase HPLC (1200 Infinity Series, Agilent Technologies) equipped with a C18 column. Cy5.5-GFLGGKGG-COOH was obtained as a result.

For synthesis of Cy5.5-GFLGGK(BHQ-3)GG-COOH, the Cy5.5-GFLGGKGG-COOH was mixed with BHQ-3-succinimidly ester, stirred at 37 °C for 2 hours, and then purified by reversed-phase HPLC. Subsequently, it was conjugated with EGF for synthesis of EGF-probe. Briefly, the Cy5.5-GFLGGK(BHQ-3)GG-COOH was mixed with EDC and NHS in PBS (pH 6.0) and stirred for 1 hour. After adding EGF, the reaction mixture was stirred further at 37 °C for 6 hours. Then, the mixture was purified using a disposable PD10 desalting column. The prepared EGF-probe was stored at 4 °C prior to use.

### Preparation Example 4. Synthesis of C-prodrug

(Ac)CGFLG, N-hydroxysuccinimide and ethyl-3-(3-dimethylaminopropyl)carbodiimide were dissolved in DMSO and the reaction mixture was stirred at 37 °C for 1 hour. After adding DOX, DIPEA was added to remove DOX hydrochloride. The reaction mixture was stirred at 37 °C for 4 hours. (Ac)CGFLG-DOX C-prodrug was obtained by purifying the reaction mixture by reversed-phase HPLC and stored after freeze-drying.

### Preparation Example 5. Synthesis of EGF-prodrug

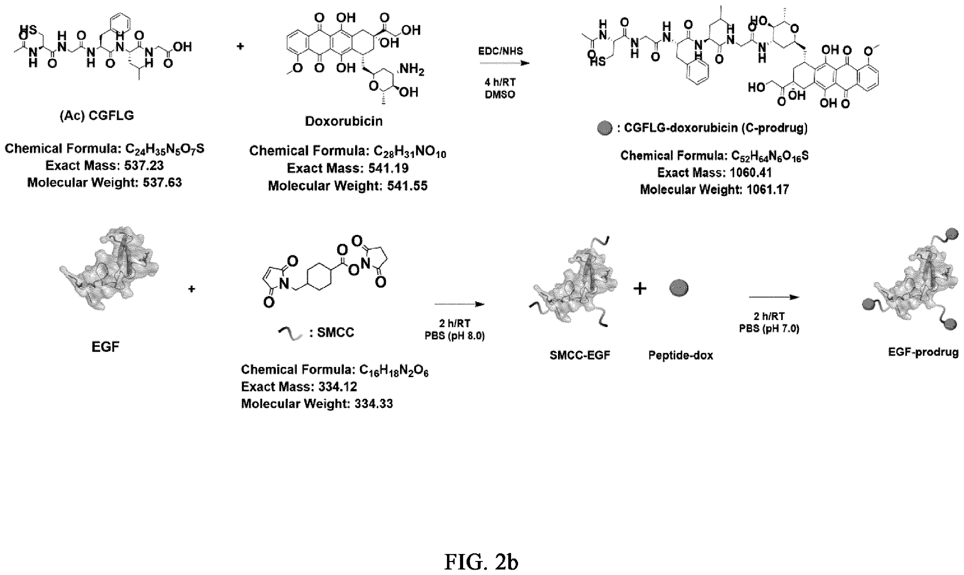

EGF-prodrug was synthesized according to FIG. 2b. EGF-prodrug was prepared by conjugating SMCC (sulfosuccinimidyl-trans-4-(N-maleimidomethyl)cyclohexane-1-carboxylate)-bound EGF with C-prodrug as follows.

First, (Ac)CGFLG, N-hydroxysuccinimide and ethyl-3-(3-dimethylaminopropyl)carbodiimide were dissolved in DMSO and the reaction mixture was stirred at 37 °C for 1 hour. After adding DOX, DIPEA was added to remove DOX hydrochloride. The reaction mixture was stirred at 37 °C for 4 hours. (Ac)CGFLG-DOX C-prodrug was obtained by purifying the reaction mixture by reversed-phase HPLC.

For preparation of SMCC-EGF, EGF and SMCC were dissolved in distilled water and then reacted at 37 °C for 6 hours. The reaction mixture was purified using a PD10 desalting column. The synthesized SMCC-EGF was mixed with C-prodrug in PBS (pH 8.0) and then purified using a PD10 column. The EGF-prodrug was recovered and stored at 4 °C prior to use.

### Test Example 1. Characterization of EGF-prodrug and EGF-probe 1

The EGF-probe of Preparation Example 3 and the EGF-prodrug of Preparation Example 5 were analyzed using a Cary 300 spectrophotometer (Agilent Technologies), a Quantamaster 40 spectrofluorometer (Horiba Scientific, Kyoto, Japan), an analytical reversed-phase HPLC system equipped with a MAbPac^{™} RP (3.0 × 100 mm) column and an ISQ^{™} EM single quadrupole mass spectrometer (Thermo Fisher Scientific, IL, USA). The molecular weight of the C-prodrug of Preparation Example 4, GFLG-DOX (m/z: [M+H]⁺ calcd for C₅₂H₆₄N₆O₁₆S 1062.17, Found 1062.5) was measured by mass spectrometer. The EGF, C-probe (Preparation Example 2), EGF-probe (Preparation Example 3), C-prodrug (Preparation Example 4) and EGF-prodrug (Preparation Example 5) were characterized by analytical reversed-phase HPLC. Specific conditions were as follows: 0-100% acetonitrile containing 0.1% TFA versus distilled water containing 0.1% TFA, flow rate = 0.3 mL/min, 22 minutes.

FIGS. 3a-3d show the HPLC analysis results. FIG. 3a shows the HPLC analysis result for EGF, FIG. 3b shows the HPLC analysis result for the C-probe of Preparation Example 2, FIG. 3c shows the HPLC analysis result for the EGF-probe of Preparation Example 3, and FIG. 3d shows the HPLC analysis result for a mixture of EGF and the EGF-probe. The signals were measured at 254 nm.

As shown in FIGS. 3a-3d, the EGF-probe of Preparation Example 3 and the EGF-prodrug of Preparation Example 5 were analyzed first by analytical reversed-phase HPLC: EGF (9.95 min), C-probe (Preparation Example 2, 11.53 min), EGF-probe (Preparation Example 3, 12.07 min). Through this, it was identified that the EGF-probe was synthesized successfully through conjugation of EGF and C-probe.

FIGS. 4a-4d show the HPLC analysis results. FIG. 4a shows the HPLC analysis result for EGF, FIG. 4b shows the HPLC analysis result for the C-prodrug of Preparation Example 4, FIG. 4c shows the HPLC analysis result for the EGF-prodrug of Preparation Example 5, and FIG. 4d shows the HPLC analysis result for a mixture of EGF and the EGF-prodrug. The signals were measured at 254 nm.

As shown in FIGS. 4a-4d, EGF, C-prodrug (10.28 min) and EGF-prodrug (11.31 min) were identified, confirming successful conjugation of the C-prodrug and EGF.

FIG. 5 shows the mass spectrum of the C-prodrug confirming the molecular weight of the C-prodrug.

### Test Example 2. Characterization of EGF-prodrug and EGF-probe 2

UV-vis absorption spectra were obtained using a spectrophotometer. Photoluminescence emission spectra were obtained using a spectrofluorometer. The relative photoluminescence quantum yield (PLQY) was measured in PBS using DOX ((Φ_{F}) of 0.04) and cyanine-5.5 ((Φ_{F}) of 0.23). The size distribution of the EGF-probe of Preparation Example 3 and the EGF-prodrug of Preparation Example 5 in PBS was analyzed using Zetasizer Nano ZS (Malvern Panalytical, Malvern, UK) by DLS (dynamic light scattering).

For evaluation of lysosomal enzyme-specific cleavage of the EGF-probe of Preparation Example 3 and the EGF-prodrug of Preparation Example 5, they were incubated with papain at various concentrations in PBS at 37 °C for 1 hour. The quenching and dequenching of the EGF-probe of Preparation Example 3 was measured using a spectrophotometer, a spectrofluorometer and an IVIS fluorescence imaging system (Lumina series III, Perkin Elmer, MA, USA). The cleavage profile of the EGF-prodrug of Preparation Example 5 was obtained by analytical reversed-phase HPLC.

FIGS. 6a-6f show the result of analyzing the characteristics of the EGF-probe and the EGF-prodrug. FIG. 6a shows the size distribution of the EGF-probe, FIG. 6b shows the fluorescence intensity of the EGF-probe, FIG. 6c shows the photoluminescence quantum yield (PLQY) of the EGF-probe in the presence of papain of various concentrations, FIG. 6d shows the size distribution of the EGF-prodrug, FIG. 6e shows the HPLC chromatograms of the EGF-prodrug, DOX, G-DOX and EGF, and FIG. 6f shows the HPLC chromatograms of the EGF-prodrug in the presence of papain at various concentrations.

As shown in FIG. 6a, the average hydrodynamic size of the EGF-probe measured by dynamic light scattering (DLS) was 75.25 ± 17.09 nm.

As shown in FIG. 6b and FIG. 6c, the EGF-probe was treated with papain of various concentrations at 37 °C for 1 hour. The signal intensity and photoluminescence quantum yield (PLQY) of the EGF-probe were increased up to 0.1 U/mL.

As shown in FIG. 6d, the average size of the EGF-prodrug was 88.97 ± 10.62 nm.

As shown in FIG. 6e and FIG. 6f, the HPLC peaks of the EGF-prodrug, DOX, G-DOX and EGF were analyzed further to define the HPLC peak of G-DOX cleaved from the EGF-prodrug. It was confirmed that, when treated with papain (1 U/mL) for 1 hour, the EGF-prodrug was cleaved into G-DOX 100%.

### Test Example 3. Evaluation of cellular uptake and fluorescence activity of EGF-probe in vitro

HCT-15, HT-29, Lovo and HCT-8 (human colorectal adenocarcinoma cell line) cells were acquired from Korean Cell Line Bank (Seoul, Republic of Korea) and cultured in an RPMI 1640 medium (GenDEPOT, TX, USA) supplemented with 10% fetal bovine serum (FBS) (GenDEPOT) and 1% antibiotics (streptomycin, 100 U/mL penicillin) (GenDEPOT).

In order to obtain fluorescence images in vitro, the HCT-15 cells (3×10⁵ cells/dish) were plated on a 35-mm confocal culture dish and cultured overnight. Next day, the cells were cultured with the EGF-probe of Preparation Example 3 (10 µg/mL) and incubated for 4 hours. For staining lysosomes, the cells were treated with LysoTracker Green (1 µM) for 1 hour before removing the EGF-probe. Then, the cells were washed 3 times with PBS, fixed with 4% paraformaldehyde and observed with a TCS SP8 confocal laser microscope (Leica, Wetzlar, Germany). The fluorescence profiles of Cy5.5 and LysoTracker Green were obtained using the LasX software. For evaluation of the recovery of EGFR-based endocytosis and lysosome-specific fluorescence of the EGF-probe, the cells were co-treated with 1 mg/mL cetuximab or 1 nM bafilomycin A1 (BafA). After incubation for 4 hours, followed by staining of cell nuclei with DAPI, the cells were observed with a confocal microscope. Flow cytometry was conducted using a Guava easyCyte HT flow cytometer (Millipore, MA, USA) and the data were analyzed with the FlowJo software. In order to compare the efficiency of fluorescence recovery for various colorectal cancer cells, HCT-15, HT29, Lovo and HCT-8 cells were plated on a confocal dish or a 24-well plate and treated with the EGF-probe for 4 hours. The fluorescence images of the four different cancer cells were acquired using a confocal microscope. For quantification of fluorescence intensity, the cells were lysed with a RIPA buffer and the whole cell lysate was transferred to a 96-well black plate for IVIS fluorescence imaging. Images were acquired at 660 nm (excitation) and 710 nm (emission). The fluorescence intensity of each well was measured using the Living Image software.

### Test Example 3. Fluorescence imaging analysis of EGF-prodrug in vitro

In order to investigate how the EGF-prodrug prepared in Preparation Example 5 works in cells in real time, HCT-15 cells were treated with 10 µM EGF-prodrug and the cells were analyzed with a confocal microscope 0.5 hour, 4 hours and 24 hours later. LysoTracker Green was added 0.5 hour before removing the medium containing the EGF-prodrug. That is to say, after pretreatment for 0.5 hour, HCT-15 cells were co-treated with the EGF-prodrug and LysoTracker Green. After a predetermined time, the cells were washed and fixed with 4% paraformaldehyde. Then, immunocytochemistry was performed to label EGFR. The fixed cells were blocked with 1% (w/v) bovine serum albumin for 1 hour and then incubated with anti-EGFR antibody at room temperature for 2 hours. After intensive washing, the cells were incubated with Alexa Fluor 647-conjugated goat anti-rabbit IgG secondary antibody at room temperature for 1 hour. After staining nuclei with DAPI, the cells were observed with a confocal microscope. The location of DOX in the cells was analyzed quantitatively using the ImageJ software. For evaluation of the action of the EGF-prodrug in the cell upon inhibition of EGFR or lysosomal activity, HCT-15 cells were treated with cetuximab (1 mg/mL) or BafA (1 nM) and fluorescence images were obtained 4 hours and 24 hours later. For comparison of lysosomal localization of the EGF-prodrug in various colorectal cancer cells, HCT-15, HT29, Lovo and HCT-8 cells were treated with the EGF-prodrug (10 µM) and LysoTracker (1 µM) and then observed with a confocal microscope 4 hours later. Then, the nuclear translocation of DOX cleaved from the EGF-prodrug in the HGF-15, HT29, Lovo and HCT-8 cells was measured 24 hours after the treatment of the EGF-prodrug.

FIGS. 7a-7g show a result of observing the recovery of specific fluorescence of the EGF-probe self-quenched in vivo or in vitro. FIG. 7a shows the result of observing the co-localization of lysosomes (green) and Cy5.5 (red) from the EGF-probe in the HCT-15 colorectal cancer cells treated with the EGF-probe for 4 hours. The lysosomes were stained with LysoTracker Green. The fluorescence intensity profiles of Cy5.5 and the lysosomes (right) were determined within the lines marked in the confocal microscopic images (left). Bar = 50 µm.

Specifically, it was evaluated whether the EGF-probe has the ability of intracellular localization and fluorescence recovery in vitro. It was confirmed that the dark-quenched EGF-probe can quickly recover Cy5.5 fluorescence after papain was added. Subsequently, it was investigated whether the EGF-probe can be dequenched also in the lysosomes of cells. For this, HCT-15 human colorectal cancer cells were treated with the EGF-probe and cultured for 4 hours. At the same time, lysosomes were stained using LysoTracker Green. Then, the cells were observed with a confocal microscope.

As shown in FIG. 7a, the EGF-probe showed strong Cy5.5 fluorescence in the HCT-15 cell. It can be seen that many fluorescence signals overlap with the lysosome signals.

FIG. 7b shows the confocal microscopic images of the HCT-15 cells treated with the EGF-probe and cetuximab (EGFR inhibitor) or with the EGF-probe and BafA (lysosome inhibitor) (DAPI: nuclei, bar = 50 µm).

As shown in FIG. 7b, a high level of co-localization of Cy5.5 and lysosomes was confirmed through the analysis of fluorescence intensities. In order to investigate the EGFR-mediated cellular uptake of the EGF-probe, HCT-15 cells were treated with the anti-EGFR antibody cetuximab (0.5 mg/mL) that can inhibit EGFR. Cetuximab blocks binding to the EGFR ligand and is used to evaluate the EGFR-mediated cellular uptake of EGF-based nanoparticles.

In addition, in order to investigate whether the dequenching of the EGF-probe depends on the lysosomal activity of cancer cells, cells were treated with 10 nM bafilomycin A1 (BafA) and a specific inhibitor of vacuolar type H(+)-ATPase at the same time. Treatment with BafA can inhibit lysosomal degradation by increasing pH in lysosomes. Several lysosomal enzymes can exhibit maximum activity at acidic pHs. It is known that the optimum pH conditions of lysosomal enzymes such as hydrolases and cathepsin B are 4.5-5.5 and they lose activity at neutral pH conditions.

FIG. 7c shows the flow cytometry analysis result of HCT-15 cells treated with the EGF-probe and cetuximab (EGFR inhibitor) or with the EGF-probe and BafA (lysosome inhibitor).

As shown in FIG. 7c, the Cy5.5 fluorescence signal was decreased remarkably when the cells were co-treated with the EGF-probe and cetuximab or BafA. From the flow cytometric analysis, it can be seen that fluorescence was decreased significantly in the group treated with cetuximab and BafA.

FIG. 8 shows the confocal microscopic images of HCT-15 cells treated with the C-probe or the EGF-probe (10 µg/mL) for 2 hours.

As shown in FIG. 8, the EGF-probe showed enhanced fluorescence signals in the HCT-15 cell as compared to the C-probe not including EGF. Through this, it can be seen that the introduction of the EGF ligand allows effective penetration also into EGFR-expressed colorectal cancer cells.

### Test Example 4. Fluorescence imaging analysis of EGF-probe and EGF-prodrug

All experiments with living animals were conducted in compliance with the relevant laws and institutional guidelines of the Institutional Animal Care and Use Committee (IACUC) of the Korea Institute of Science and Technology (KIST) and approved by the IACUC (approval number: 2019-092).

The biodistribution of the EGF-probe intravenously administered to mice having HCT-15, HT29, Lovo or HCT-8 tumors was evaluated. First of all, 6-week-old Balb/c- nude mice (Nara-Biotec, Gyeonggi-do, Korea) were prepared and each cell was subcutaneously inoculated into the nude mice at the right flank at a concentration of 2×10⁶ cells/mouse to generate tumors. When the tumor volume reached ~150 mm³, the EGF-probe (5 mg/kg) was injected into the tail vein. At 24 hours after the administration of the EGF-probe, the mouse was imaged using an IVIS fluorescence imaging system. To compare the potential use of EGF-Cy5.5, the C-probe and the EGF-probe for tumor-specific imaging, HCT-15 tumor-bearing mice were intravenously injected with EGF-Cy5.5, the C-probe or the EGF-probe. IVIS fluorescence imaging was conducted at 24 hours after the administration. Major organs and tumors were obtained and imaged with an IVIS imaging system. The fluorescence intensity of the organs and tumors was analyzed using the Living Image 2.50 software. The collected tumor tissues were embedded in the OCT compound and then cryosectioned for observation of Cy5.5 fluorescence. The tumor tissue sections were counter-stained with DAPI and visualized with a confocal microscope.

In addition, the tumor-targeting ability of DOX, the C-prodrug and the EGF-prodrug was compared. Briefly, HCT-15 tumor-bearing mice were imaged 6 hours after intravenously injecting DOX, the C-prodrug or the EGF-prodrug. HCT-15- and HCT-8-bearing mice were used to investigate the tumor-specific accumulation of the EGF-prodrug.

FIG. 7d shows the IVIS images obtained 24 hours after intravenously administering EGF-Cy5.5, the C-probe or the EGF-probe to the HCT-15-bearing mice. The black arrows indicate HCT-15 tumor cells. As a result of in-vivo fluorescence imaging at 24 hours after the injection of EGF-Cy5.5, the C-probe or the EGF-probe (5 mg/kg) to the HCT-15-bearing mice, EGF-Cy5.5 showed a strong fluorescence signal at the tumor site but nonspecific background signals were observed throughout the body when compared with the C-probe and the EGF-probe.

FIG. 7e shows the fluorescence images of various organs obtained 24 hours after intravenously administering EGF-Cy5.5, the C-probe or the EGF-probe to the HCT-15-bearing mice (H: heart, Lu: lung, Li: liver, Sp: spleen, Ki: kidney, Tu: tumor). FIG. 7f shows the fluorescence intensity of various organs obtained from the HCT-15-bearing mice treated with EGF-Cy5.5, the C-probe or the EGF-probe (left) and the fluorescence intensity of tumors divided by the fluorescence intensity of the total organs (right). n = 3 per group, ns: nonsignificant. **P < 0.01 and ***P < 0.001.

Due to the presence of the fluorescence switching system and the EGF ligand, precise and distinct tumor-specific fluorescence signals and lowest background signals were observed for the EGF-probe. As a result of observing fluorescence signals from the major organs of the mice treated with EGF-Cy5.5, the C-probe or the EGF-probe, the EGF-probe showed high fluorescence intensity comparable to EGF-Cy5.5 in tumors but the fluorescence signal was hardly detected in the lung, liver, spleen, etc. In order to investigate the ability of EGF-Cy5.5, the C-probe or the EGF-probe for tumor-specific imaging, the fluorescence intensity of tumors was divided by the sum of the fluorescence intensities of all the major organs. The EGF-probe showed a tumor-specific fluorescence intensity ratio increased by 2.8 times and 3.3 times as compared to the EGF-Cy5.5 and the C-probe, respectively.

FIG. 7g shows the fluorescence images of tumor tissues obtained from mice to which the GF-Cy5.5, the C-probe or the EGF-probe was administered. Bar = 50 µm.

As shown in FIG. 7g, whereas the tumor tissues were observed clearly when the EGF-Cy5.5 or the EGF-probe was treated, much weaker fluorescence signals were observed for the C-probe as compared to the EGF-Cy5.5 or the EGF-probe.

To summarize, it was confirmed that the internalization of the EGF-probe in cancer cells is mediated by EGFR and the fluorescence dequenching of the internalized EGF-probe is induced by lysosomes, particularly lysosomal enzymes. Tumor-targeting efficiency is improved greatly through conjugation with the EGF ligand, and this allows accurate and specific tumor cell imaging by remarkably reducing background signals.

FIGS. 9a-9d show a result of investigating the EGFR-mediated internalization and specific cleavage of DOX from the EGF-prodrug. FIG. 9a shows the time-lapse images of DOX (red) in HCT-15 cells treated with the EGF-prodrug. Lysosomes (green) were stained for 30 minutes before removing the EGF-prodrug. EGFR: gray, lysosomes: green, nuclei: blue arrows. Bar = 20 µm.

As shown in FIG. 9a, unlike the EGF-probe, fluorescence was detected from the lysosomes for the EGF-prodrug after quenching and the EGF-prodrug was converted continuously to DOX. In order to determine the intracellular fate of the EGF-prodrug in colorectal cancer cells where EGFR is expressed, HCT-15 cells were treated with the EGF-prodrug and confocal microscopic images were acquired 0.5 hour, 4 hours and 24 hours later.

FIG. 9b shows a result of analyzing the intracellular localization of DOX at 0.5 hour, 4 hours and 24 hours after treatment with the EGF-prodrug (N: nuclei, E: EGFRs, L: lysosomes). Bar = 20 µm.

As shown in FIG. 9b, it was confirmed clearly that the EGF-prodrug can bind to EGFR. The intracellular localization of the EGF-prodrug was confirmed after 4 hours. The EGF-prodrug could be cleaved to DOX by lysosomal enzymes present in the lysosomes and, as a result, the fluorescence signals of DOX were observed clearly in the cell nuclei with the lapse of time.

FIG. 9c shows the fluorescence microscopic images of the HCT-15 cells obtained at 4 hours and 24 hours after treating with cetuximab or BafA and the EGF-prodrug. Bar = 50 µm.

EGFR-mediated intracellular localization and release of DOX induced by lysosomal enzymes were confirmed as shown in FIG. 9c. The mechanism of the EGF-prodrug when the activity of EGFR and lysosomes was inhibited using cetuximab and BafA, respectively, was investigated further.

Similarly to the EGF-probe, the inhibition of EGFR substantially decreased the uptake of the EGF-prodrug. The inhibition of lysosomal activity by treatment of BafA did not affect the cellular uptake of the EGF-prodrug, but the nuclear translocation of DOX was decreased significantly.

### Test Example 5. In-vitro toxicity test of EGF-prodrug

The cytotoxicity of the EGF-prodrug of Preparation Example 5 was investigated. In addition, it was investigated whether the cytotoxicity of the EGF-prodrug is attenuated by BafA. First, HCT-15 cells were plated on a 96-well plate at a density of 5×10³ cell/well. Then, the cells were incubated with the EGF-prodrug (0-100 µM). 48 hours later, the medium was removed and the cells were treated with a cell counting kit (CCK-8, Dojindo, Japan) at 37 °C for 1 hour. Absorbance was measured at 450 nm using a VERSAmax^{™} microplate reader (Molecular Devices Corp., CA, USA). The cell viability of each group (n = 5) was represented as a percentage of normal untreated cells (control group). For comparison of the cytotoxicity of the EGF-prodrug and DOX in four different colorectal cancer cells, HCT-15, HT29, Lovo and HCT-8 cells were incubated with 0.001-50 µM of the EGF-prodrug or DOX at 37 °C for 48 hours. After removing the medium, cell viability was evaluated by CCK-8 assay.

FIG. 9d shows the result of measuring cytotoxicity after treating HCT-15 cells with the EGF-prodrug (0, 10 and 100 µM) or BafA (0, 0.1 and 1 nM) at various concentrations. n = 5, * P < 0.05, *** P < 0.001.

As shown in FIG. 9d, cell viability was increased gradually with concentration when the cells were co-treated with the EGF-prodrug and BafA. It can be seen that the inhibition of lysosomal activity can lead to inhibition of the release of DOX from the EGF-prodrug and significantly reduce the toxicity of the EGF-prodrug for cancer cells.

### Test Example 5. Western blot assay

The phosphorylation of AKT and ERK1/2 was measured by western blot assay. HCT-15 cells were plated on a 6-well plate at a density of 5×10⁵ cells/well. After stabilization, the cells were treated with EGF, the EGF-probe or the EGF-prodrug of the same concentration of EGF (50 ng/mL) for 20 minutes. After washing the cells immediately with PBS, total cell lysate was collected using a RIPA buffer supplemented with a protease/phosphatase inhibitor cocktail. After incubation on ice for 1 hour, the lysate was centrifuged at 14,000 g for 20 minutes. After the centrifugation, the supernatant was collected and subjected to BCA assay for measurement of protein concentration. The proteins were loaded onto 8% (w/v) SDS-polyacrylamide gel. After conducting electrophoresis at 80 V for 100 minutes, the proteins were transferred to a nitrocellulose membrane (Bio-Rad, CA, USA) at 25 V for 30 minutes using a Trans-Blot^{™} Turbo transfer system. Then, after blocking the membrane for 2 hours using 5% (w/v) skim milk, the proteins were probed with primary antibodies at 4 °C for 16 hours. Then, after intensively washing the blots with TBS-T, they were incubated with HRP-conjugated goat anti-rabbit secondary antibodies or HRP-conjugated horse anti-mouse secondary antibodies at room temperature for 1 hour. The captured proteins were developed using a chemiluminescence detection system and observed with an iBright FL1000 imaging system (Invitrogen, CA, USA).

FIG. 10 shows a result of measuring EGFR expression level in HCT-15, HT29, Lovo and HCT-8 cells by western blot (n = 3). As shown in FIG. 10, the EGFR expression level was highest in the Lovo cells, moderate in the HCT-15 and HCT-8 cells, and lowest in the HT29 cells.

FIGS. 11a-11e show a result of investigating the relationship between the fluorescence recovery of the EGF-probe and the cytotoxicity of the EGF-probe in HCT-15, HT29, Lovo and HCT-8 cells. FIG. 11a shows fluorescence images obtained at 4 hours after treating HCT-15, HT29, Lovo and HCT-8 cells with EGF-probe. Bar = 50 µm.

As shown in FIG. 11a, the EGF-probe was observed vaguely in the Lovo and HCT-8 cells wherein the EGFR expression level was the lowest or highest, but distinct fluorescence signals of the EGF-probe were observed in other cells.

FIG. 11b shows the IVIS fluorescence images of cell lysates obtained by treating HCT-15, HT29, Lovo and HCT-8 cells with EGF-probe at various concentrations (top) and a result of quantifying fluorescence signals (bottom). n = 3, *** P < 0.001.

As shown in FIG. 11b, the total cell lysates of HCT-15, HT29, Lovo and HCT-8 cells were imaged with IVIS and their fluorescence intensities were measured. Whereas the HCT-15 and HT29 cells exhibited strong fluorescence signals, the Lovo and HCT-8 cells showed weak fluorescence signals. Although high fluorescence intensity was expected in the cells with high EGFR expression levels because the intracellular localization of the EGF-probe is mediated by EGFR, there was no significant correlation between the EGFR expression level and the fluorescence signal intensity of the EGF-probe.

In addition, the signal detection intensity was increased with the concentration of the EGF-probe. That is to say, the optimum concentration of the EGF-probe for obtaining superior in all tumor cells was 20 µg/mL.

FIG. 11c shows the in-vivo IVIS images of mice in which HCT-15, HT29, Lovo and HCT-8 cells were transplanted obtained after administering the EGF-probe (5 mg/kg). The graph in the right side of FIG. 11c shows a result of quantitatively measuring fluorescence intensity from the fluorescence images of FIG. 11c using the Living Image software. n = 4 per group, ** P < 0.01 and P < 0.001.

As shown in FIG. 11c, after injecting the EGF-probe to mice to which HCT-15, HT29, Lovo or HCT-8 cancer cells were administered, IVIS fluorescence images were acquired 24 hours later. As a result, it was confirmed that the EGF-probe showed significantly high fluorescence intensity at the tumor sites of the mice, but the fluorescence signals were hardly observed in other areas.

FIG. 11d shows a result of measuring cytotoxicity after treating HCT-15, HT29, Lovo and HCT-8 cells with the EGF-prodrug and free DOX at various concentrations (n = 5). FIG. 11e shows the IC₅₀ ratio of the EGF-prodrug versus free DOX in the HCT-15, HT29, Lovo and HCT-8 cells.

As shown in FIG. 11d and FIG. 11e, cell viability was measured after treating HCT-15, HT29, Lovo and HCT-8 cells with the EGF-prodrug (0.1-50 µM) DOX) or free DOX (0.01-50 µM). It was confirmed that cytotoxicity was increased with the concentration of the EGF-prodrug and DOX. The EGF-prodrug showed lower cytotoxicity in the concentration range of 0.1-5 µM than free DOX. At concentrations above 10 µM, the EGF-prodrug and DOX showed similar cytotoxicity. The EGF-prodrug showed the lowest IC₅₀ ratio in the HCT-15 cells and the highest IC₅₀ ratio in the HCT-8 cells.

It was confirmed that the fluorescence intensity and cytotoxicity of the EGF-prodrug had significant correlation in the four cancer cells. It was investigated whether the EGF-probe can be used to predict the anticancer effect of the EGF-prodrug.

The introduction of the EGF ligand can stimulate cancer cells by activating the EGFR signaling pathway. Therefore, it was investigated whether the EGF-probe or the EGF-prodrug induces phosphorylation of EGFR and ERK1/2.

FIG. 12 shows a result of treating HCT-15 cells with PBS (control group), EGF, EGF-probe and EGF-prodrug (50 ng/mL of EGF) for 20 minutes and investigating phosphorylation of EGFR and ERK1/2.

As shown in FIG. 12, the phosphorylation of EGFR and ERK1/2 was not increased in the HCT-15 cells treated with the EGF-probe or the EGF-prodrug (50 ng/mL of EGF) as compared to EGF (50 ng/mL) as a positive control group. Through this, it can be seen that an EGF-conjugated nanocarrier can penetrate into colon cancer cells without activation of EGFR, unlike free EGF.

### Test Example 6. Analysis of antitumor effect of EGF-prodrug in vivo

2×10⁶ HCT-15 cells or HCT-8 cells were subcutaneously inoculated to Balb/c-nude mice. When the tumor volume reached 150-200 mm³, the mice were randomly divided into four groups; PBS injection (control group), free DOX, C-prodrug and EGF-prodrug. DOX, the C-prodrug and the EGF-prodrug were injected at a dosage of 1 mg/kg based on DOX. The injection was carried out into the tail vein every 3 days for a total of 6 times. Antitumor effect was evaluated by sacrificing the mice to which the HCT-15 or HCT-8 cells were administered and measuring the volume of tumor tissue. The volume of the tumor tissue was calculated every day or every other day by 0.53 × length × width². Tumor growth was monitored for 21 days. After sacrificing the mice, the HCT-15 or HCT-8 tumor tissues were collected. The dissected tumors were imaged and fixed by treating with 4% paraformaldehyde. After embedding in paraffin, the tumor tissues were prepared into 10 µm-thick sections. The sections were used for histological or pathological investigation. They were stained with H&E for visualization and subjected to TUNEL assay using a DeadEND^{™} TUNEL system (Promega, WI, USA) for investigation of the apoptosis of cancer cells. The tumor tissues stained with H&E and TUNEL were observed using a BX51 optical microscope (Olympus, Tokyo, Japan) and a TCS SP8 confocal laser microscope.

The positive correlation between the fluorescence recovery and cytotoxicity of the EGF-prodrug in tumor cells was investigated. Because there was no significant correlation of EGFR expression level between the EGF-probe and the EGF-prodrug in the experiment described above, the level of lysosomal activity in HCT-15, HT29, Lovo and HCT-8 cells was analyzed.

FIGS. 13a-13g show a result of analyzing the mechanism of the EGF-prodrug for various colorectal cancer cells in vitro. FIG. 13a shows a result of analyzing the intracellular expression level of EGFR and LAMP-1 in HCT-15, HT29, Lovo and HCT-8 cells by western blot.

As shown in FIG. 13a, as a result of treating various colorectal cancer cells with LysoTracker Green for 1 hour and conducting flow cytometric analysis, distinct histogram shift of LysoTracker Green was observed in the HCT-15 and HT29 cells, but insignificant histogram shift was observed in the Lovo and HCT-8 cells.

FIG. 13b shows a result of analyzing rectal cancer cells treated with LysoTracker Green by flow cytometry. The cells of the control group were treated with nothing.

As shown in FIG. 13b, the numbers of HCT-15 and HT29 cells stained with LysoTracker Green was significantly larger than Lovo and HCT-8 cells.

FIG. 13c shows confocal microscopic images obtained at 4 hours after treating HCT-15, HT29, Lovo and HCT-8 cells with the EGF-prodrug. They indicate the degree of lysosomal localization of the EGF-prodrug in the HCT-15, HT29, Lovo and HCT-8 cells. Bar = 10 µm. The confocal microscopic images of the cancer cells were analyzed after incubating them with the EGF-prodrug for 4 hours and then staining with LysoTracker Green.

As shown in FIG. 13c, lysosomes and DOX were co-localized in the HCT-15 and HT29 cells.

FIG. 13d shows a result of measuring fluorescence intensity at 4 hours after treating HCT-15, HT29, Lovo and HCT-8 cells with the EGF-prodrug to investigate the co-localization efficiency of DOX (red) and lysosomes (green). The graphs were obtained from confocal images. FIG. 13e shows the correlation between the fluorescence intensity of DOX and lysosomes. The dotted red lines indicate fitting of the linear regression curves. n = 8, #P < 0.05 versus HCT-15, §P < 0.05 versus HT-29.

As shown in FIG. 13d and FIG. 13e, high level of co-localization was observed in the HCT-15 and HT29 cell, but insignificant co-localization was observed in the Lovo and HCT-8 cells. In addition, whereas the HCT-15 and HT29 cells showed strong linear spatial correlation between DOX and lysosomes, significantly weaker correlation was observed for the Lovo and HCT-8 cells. The lysosomal localization of the EGF-prodrug and the separation of DOX from the EGF-prodrug are essential for the transport of DOX to cell nuclei.

FIG. 13f shows fluorescence images obtained at 24 hours after treating HCT-15, HT29, Lovo and HCT-8 cells with the EGF-prodrug. The localization of DOX can be observed directly. Bar = 50 µm. FIG. 13g shows a result of quantifying the ratio of co-localization of DAPI (cell nuclei) with DOX. n = 5, #P < 0.05 versus HCT-15, §P < 0.05 versus HT-29.

As shown in FIG. 13f, the quantity of DOX accumulated in cell nuclei was observed at 24 hours after treating with the EGF-prodrug of the present disclosure. The fluorescence signals of DOX localized in the nuclei of the HCT-15 and HT29 cells in large quantities were confirmed from confocal microscopic images. This was verified by the quantitative analysis of DOX co-localized with DAPI (nuclei). Specifically, it was confirmed that 91-100% of co-localization was accomplished in the HCT-15 and HT29 cells. In contrast, the accumulation of DOX in the nuclei of the Lovo and HCT-8 cells was remarkably less, which may be due to the low cytotoxicity of the EGF-prodrug in these cells.

Because tumor-targeting ability is improved by the binding of the self-quenching probe and the EGF ligand, it was investigated whether the accumulation of a tumor-specific prodrug in the body is also improved by the introduction of the EGF ligand.

FIGS. 14a-14h show a result of evaluating the anticancer effect and tumor-targeting ability of the EGF-prodrug in vivo. FIG. 14a shows the IVIS fluorescence images of HCT-15-bearing mice at 6 hours after administration of DOX (5 mg/kg), the C-prodrug (5 mg/kg of DOX) or the EGF-prodrug (5 mg/kg of DOX).

As shown in FIG. 14a, the C-prodrug or the EGF-prodrug (5 mg/kg of DOX) was intravenously administered to HCT-15-bearing mice and IVIS fluorescence images were acquired 6 hours later. As a result, the strongest fluorescence signal was observed at the tumor site of the mouse to which the EGF-prodrug was injected.

FIG. 14b shows fluorescence images showing the ex-vivo distribution of intravenously administered DOX, C-prodrug or EGF-prodrug (H: heart, Lu: lung, Li: liver, Sp: spleen, Ki: kidney, Tu: tumor). The images were acquired at 24 hours after the administration.

As seen from the ex-vivo distribution of DOX, the C-prodrug and the EGF-prodrug shown in FIG. 14b, the EGF-prodrug was specifically and remarkably accumulated in tumor tissues when compared with the C-prodrug.

When DOX was administered alone, significant amounts were accumulated also in the lung, liver and kidney, in addition to tumor tissue. In contrast, the EGF-prodrug was accumulated highly in tumor tissue and relatively small amounts were accumulated in other organs.

FIG. 14c shows the fluorescence intensity of various organs (left) and the fluorescence intensity of tumors divided by the fluorescence intensity of the total organs (right). n = 3 per group, *P < 0.05, **P < 0.01.

As shown in FIG. 14c, the EGF-prodrug was accumulated tumor-specifically the most precisely and effectively.

FIG. 14d shows the fluorescence images of tumor tissues isolated from mice to which DOX, the C-prodrug or the EGF-prodrug was administered. Bar = 50 µm.

As shown in FIG. 14d, a considerable amount of the EGF-prodrug was accumulated in the tumor tissue.

FIG. 14e shows a result of injecting DOX, the C-prodrug or the EGF-prodrug (1 mg/kg of DOX, 6 times every 3 days) to HCT-8- or HCT-15-bearing mice and measuring tumor size for 21 days. PBS was injected to the control group. n = 4 per group, *P < 0.05, **P < 0.01.

As a result of injecting the DOX, C-prodrug or EGF-prodrug to HCT-8- or HCT-15-bearing mice 6 times every 3 days and measuring tumor size for 21 days, the tumor size was not controlled significantly in the HCT-8-bearing mice after the administration of the DOX, C-prodrug or EGF-prodrug, as shown in FIG. 14e. However, in the HCT-15-bearing mice, the tumor growth was significantly when the EGF-prodrug was administered as compared to the control group, DOX or the C-prodrugs.

FIG. 14f shows tumor tissues taken from HCT-8- or HCT-15-bearing mice on day 24 after injection of PBS, DOX, the C-prodrug or the EGF-prodrug, FIG. 14g shows a result of staining tumor tissues taken from the HCT-8- or HCT-15-bearing mice on day 24 after injection of PBS, DOX, the C-prodrug or the EGF-prodrug with H&E, and FIG. 14h shows a result of staining tumor tissues taken from the HCT-8- or HCT-15-bearing mice on day 24 after injection of PBS, DOX, the C-prodrug or the EGF-prodrug with TUNEL. Bar = 1 cm.

As shown in FIG. 14f, it was confirmed that the EGF-prodrug has very superior antitumor effect against the HCT-15 cells.

As shown in FIG. 14g and FIG. 14h, serious apoptosis was observed in the tumor tissue of the HCT-15-bearing mice to which the EGF-prodrug was administered.

FIGS. 15a and 15b show a result of analyzing the in-vivo tumor-targeting ability of the EGF-prodrug in HCT-8- or HCT-15-bearing mice. FIG. 15a shows the IVIS fluorescence images of HCT-8- or HCT-15-bearing mice at 6 hours after treatment with the EGF-prodrug (5 mg/kg of DOX), and FIG. 15b shows a result of measuring the ex-vivo distribution and fluorescence intensity of the EGF-prodrug. n = 3 per group, ns: nonsignificant.

As shown in FIGS. 15a and 15b, there was no significant difference in the amount of the EGF-prodrug accumulated in the tumors of the HCT-15- or HCT-8-bearing mice.

## Claims

1. A composition for diagnosing colon cancer or predicting medicinal efficacy, comprising a complex wherein a fluorophore and a quencher are conjugated on both sides of a peptide represented by SEQ ID NO 1, which is cleaved by a lysosomal enzyme present in a tumor cell, and EGF (epidermal growth factor) is bound to the C-terminal of the peptide.

2. The composition for diagnosing colon cancer or predicting medicinal efficacy according to claim 1, wherein the fluorophore is bound to the N-terminal of the peptide, and the quencher is bound to the ε-amino group of a lysine or arginine amino acid residue of the peptide.

3. The composition for diagnosing colon cancer or predicting medicinal efficacy according to claim 2, wherein the fluorophore is any one selected from a group consisting of fluorescein, FITC (fluorescein isothiocyanate), Oregon green, Texas red, Cy2, Cy3, Cy3B, Cy3.5, Cy5, Cy5.5, Cy7, indocarbocyanine, rhodamine, oxacarbocyanine, thiacarbocyanine, merocyanine, pyridyloxazole, nitrobenzoxadiazole, benzoxadiazole, Nile red, Nile orange, acridine yellow, auramine, crystal violet and malachite green.

4. The composition for diagnosing colon cancer or predicting medicinal efficacy according to claim 2, wherein the quencher is one or more selected from a group consisting of TAMRA (6-carboxytetramethyl-rhodamine), BHQ1 (black hole quencher 1), BHQ2 (black hole quencher 2), BHQ3 (black hole quencher 3), NFQ (nonfluorescent quencher), DABCYL, Eclipse, DDQ (deep dark quencher), blackberry quencher and Iowa black.

5. The composition for diagnosing colon cancer or predicting medicinal efficacy according to claim 1, wherein the colon cancer is epidermal growth factor receptor (EGFR)-positive colon cancer.

6. The composition for diagnosing colon cancer or predicting medicinal efficacy according to claim 1, wherein the complex penetrates into a colon cancer cell and emits fluorescence as it is cleaved by an enzyme existing in the lysosome of the colon cancer cell and quenching by the fluorophore and the quencher is resolved.

7. A composition for identifying prognosis for therapeutic effect in a patient with EGFR-positive colon cancer, comprising a complex wherein a fluorophore and a quencher are conjugated on both sides of a peptide represented by SEQ ID NO 1, which is cleaved by a lysosomal enzyme present in a tumor cell, and EGF (epidermal growth factor) is bound to the C-terminal of the peptide.

8. A method for providing information for predicting response to an epidermal growth factor receptor (EGFR)-targeting drug, comprising a step of treating a sample isolated from a cancer patient with the composition for diagnosing colon cancer or predicting medicinal efficacy according to claim 1 and measuring fluorescence intensity.

9. The method according to claim 8, wherein the subject is determined as responding to the EGFR drug if the fluorescence intensity is detected.

10. A method for providing information for verifying the therapeutic effect of an anticancer drug, comprising a step of treating a colon sample isolated from a cancer patient who is taking the anticancer drug with the composition for diagnosing colon cancer or predicting medicinal efficacy according to claim 1 and measuring fluorescence intensity.

11. The method according to claim 10, wherein it is determined that the anticancer drug has no therapeutic effect for the colon cancer patient if the fluorescence intensity is detected.

12. A pharmaceutical composition for preventing or treating colon cancer, comprising:
a) a complex wherein a fluorophore and a quencher are conjugated on both sides of a peptide represented by SEQ ID NO 1, which is cleaved by a lysosomal enzyme present in a tumor cell, and EGF (epidermal growth factor) is bound to the C-terminal of the peptide; and
b) a prodrug complex wherein EGF (epidermal growth factor) and an anticancer drug are conjugated on both sides of a peptide represented by SEQ ID NO 1 or SEQ ID NO 2, which is cleaved by a lysosomal enzyme present in a tumor cell.

13. The pharmaceutical composition for preventing or treating colon cancer according to claim 12, wherein the colon cancer is EGFR-positive colon cancer.

14. The pharmaceutical composition for preventing or treating colon cancer according to claim 12, wherein, in the prodrug complex b), the peptide and the EGF are covalently bonded by a crosslinking agent.

15. The pharmaceutical composition for preventing or treating colon cancer according to claim 14, wherein the crosslinking agent is one or more selected from a group consisting of protein A, carbodiimide, N-succinimidyl S-acetylthioacetate (SATA), N-succinimidyl 3-(2-pyridyldithio)propionate (SPDP) and sulfosuccinimidyl 4-(N-maleimidomethyl)cyclohexane-1-carboxylate (Sulfo-SMCC).

16. The pharmaceutical composition for preventing or treating colon cancer according to claim 12, wherein the anticancer drug is one or more selected from a group consisting of doxorubicin, cyclophosphamide, mechlorethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cytarabine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, ixabepilone, vinblastine, vincristine, vindesine, vinorelbine, estramustine, maytansine, DM1 (mertansine), DM4, dolastatin, auristatin E, auristatin F, monomethyl auristatin E, monomethyl auristatin F and derivatives thereof.
